# DEMANDE DE BREVET EUROPEEN

(11) **EP 1 666 612 A2**
(43) Date de publication de la demande: **07.06.2006**
(21) Numéro de dépôt: 06290080.8
(22) Date de dépôt: 08.12.1999
(51) Int. Cl.: C12Q 1/68

(54) **Procédé de détermination de l'activité d'une substance mettant en oeuvre un test fonctionnel in vitro**

(30) Priorité: 08.12.1998 FR 9815488
(62) Demande divisionnaire de: 99957374.4
(71) Demandeur: Proteus S.A., 30000 Nîmes (FR)
(72) Inventeur: Bloch, Jean-François, 30000 Nimes (FR); Dupret, Daniel, 30420 Calvisson (FR); Dautel, Sandrine, 30000 Nimes (FR); Masson, Jean-Michel, 31400 Toulouse (FR); Lefevre, Fabrice, 30900 Nimes (FR)
(74) Mandataire: Breese Derambure Majerowicz

(57) **Abrégé**

La présente invention concerne un procédé de détermination de l'activité *in vitro* d'une substance mettant en oeuvre un test fonctionnel, caractérisé en ce que l'on détecte et/ou mesure, la variation d'une fonction connue correspondant à une plusieurs protéines produites in vitro, en présence et en absence de la dite substance ou soit à la substance en présence ou en absence d'une ou plusieurs protéines produites *in vitro.*

## Description

La présente invention concerne le domaine de la recherche de substances capables de modifier une fonction correspondant à une protéine ou un ensemble de protéines impliquées dans un processus biologique. Ce type de travaux, aussi désigné screening ou criblage, constitue aujourd'hui un mode de recherche empirique mais particulièrement performant de nouvelles substances, utilisé dans de nombreux laboratoires. Parmi les nombreux domaines d'applications de ces stratégies de criblage, on peut citer les exemples suivants :
- Les laboratoires pharmaceutiques disposent de bibliothèques de molécules, et recherchent celles capables d'inhiber ou de ralentir l'activité d'enzymes impliquées dans le développement de maladies génétiques ou infectieuses d'origine bactérienne ou virale.
- Suite à l'utilisation intensive des antibiotiques, la vitesse d'apparition de nouvelles résistances est actuellement plus rapide que celle de découverte de nouveaux antibiotiques. Certains laboratoires hospitaliers recherchent donc, toujours à partir de banques de molécules bien spécifiques, de nouveaux antibiotiques ou de nouveaux inhibiteurs de beta-lactamase.
- Des travaux sont également effectués pour trouver des inhibiteurs de la multiplication des microorganismes impliqués dans la corrosion biologique des canalisations ou des containers utilisés dans des procédés industriels.

La présente invention a pour objet un procédé de détermination de l'activité in vitro d'une ou plusieurs substances mettant en oeuvre un test fonctionnel consistant à détecter et/ou mesurer une variation d'au moins une fonction connue correspondant soit à une ou plusieurs protéines produites *in vitro* en présence et en absence de ladite substance, soit à la substance en présence ou en absence de protéines produites *in vitro.* Le procédé de l'invention, sera donc aussi désigné ci-après, procédé de criblage.

Ladite fonction connue correspond de préférence à des protéines exprimées *in vitro* encore appelées protéines cibles. Le procédé de l'invention vise donc à déterminer l'activité de la substance testée sur une fonction connue correspondant à une ou plusieurs protéine(s) produite(s) *in vitro.*

Toutefois, dans une autre forme de mise en oeuvre du procédé de l'invention, la fonction connue correspond à la ou aux substances testé(s). Dans ce cas, le procédé de l'invention permet de déterminer si l'activité connue de cette substance est modifiée par une ou plusieurs protéines produite(s) *in vitro.*

On entend par fonction l'activité d'une ou plusieurs protéines spécifiques encore appelées protéines cibles d'au moins un organisme ou d'au moins un processus, et que l'on détecte et/ou quantifie selon la présente invention par le biais d'un test fonctionnel. Préférentiellement, la protéine cible est une enzyme. Cette enzyme peut correspondre entre autres à la réverse transcriptase ou à la protéinase à acide aspartique du virus du Sida, à l'intéine de RecA de *Mycobacterium tuberculosis,* à une protéine de résistance à un antibiotique, ou encore à un ensemble d'enzymes impliquées dans une voie métabolique.

On entend par fonction connue, la fonction qui est analysée selon le procédé de l'invention et qui peut être détectée et/ou mesurée par un test fonctionnel.

Dans un cas particulier du procédé de l'invention, on entend par fonction, l'activité d'une ou plusieurs substances que l'on détecte et/ou mesure par un test fonctionnel.

On entend par variation de fonction la différence d'activité de la fonction mesurée en présence ou en absence de substance. Dans une seconde forme de réalisation du procédé de l'invention, la variation de fonction correspond à la différence d'activité de la substance en présence ou en absence de protéines exprimée *in vitro.*

Le test fonctionnel peut mettre en oeuvre une ou plusieurs molécules rapporteurs. La molécule rapporteur peut correspondre à toute molécule capable de révéler directement ou indirectement l'activité d'une ou plusieurs protéines cibles, il peut s'agir d'une molécule d'acide nucléique, d'une protéine, d'un peptide, tel qu'un anticorps ou un mélange d'anticorps spécifiques capables de révéler l'activité d'une protéine cible, ou tel qu'un substrat ou une cascade de substrats, dont l'un est celui d'une protéine cible.

La fonction peut correspondre à une activité enzymatique ou à une affinité. Dans le cadre de la mise en évidence d'une variation de fonction correspondant à une activité enzymatique, tout type de test fonctionnel spécifique peut être envisagé par l'homme de métier pour mettre en évidence cette variation. L'homme de métier pourra par exemple se référer à des ouvrages comme Methods In Enzymology ou Annual Review Of Biochemistry, dans lesquelles un grand nombre de méthodes de dosage d'enzymes et de préparation de substrats ont été décrites. Dans le cas de la mise en évidence d'une variation de fonction correspondant à l'affinité par exemple d'un antigène pour un anticorps, d'une protéine pour de l'ADN, d'un récepteur pour un ligand etc.... la mise en évidence d'une variation de fonction peut être réalisée par exemple par des tests tels que la fixation de ligands marqués par un isotope ou par une enzyme ou par un fluorophore, par une détection immunologique utilisant des anticorps marqués par un métal ou par une enzyme ou par un fluorophore.

On entend par organisme, tout type d'organisme ou micro-organsime, comme des virus, des bactéries, des algues, des champignons, ou tout produit contenant des acides nucléiques synthétiques ou naturels permettant l'expression d'une ou plusieurs protéines codant avantageusement pour une fonction.

On entend par processus, le développement d'une maladie, d'une infection ou de cellules par exemple cancéreuses ou de contaminants ou des mécanismes de résistances bactériennes. Ces processus peuvent avoir lieu sur un hôte ou dans un procédé industriel (agroalimentaire, traitement du papier, textile).

On entend par substance des polynucléotides, des peptides, des protéines, les ions, des molécules ou des compositions chimiques naturelles ou synthétiques, des hormones, des composés aromatiques, des anticorps, des fragments d'anticorps, des gènes, des récepteurs cellulaires, des acides aminés, des glycopeptides, des lipides, des glycolipides, des sucres, des polysaccharides, etc...., capables de modifier l'activité d'une ou plusieurs fonctions d'un organisme ou d'un processus. Ces dites substances pourront correspondre à une ou plusieurs têtes de séries. Il peut s'agir de tout agent capable de modifier la ou les fonctions comme des antiviraux, des inhibiteurs, des stimulants, des conditions physico-chimiques, des radiations ou des traitements thermiques.

Le procédé de l'invention s'applique donc tout particulièrement au criblage de substances capables de modifier l'activité d'une ou plusieurs protéines cibles ou fonction, impliquée(s) dans un processus biologique grâce à l'expression *in vitro* de la dite ou desdites protéines. Le procédé de l'invention accepte le criblage de plusieurs substances simultanément, notamment lorsqu'il s'agit de substances susceptibles d'interagir pour exprimer une fonction ou pour modifier une fonction. En conséquence, il faut entendre substance tant au singulier qu'au pluriel dans le procédé de l'invention décrit ci-après.

De même l'emploi par la suite du terme "fonction" au singulier couvrira également le terme "fonctions" au pluriel et vice versa, sauf lorsqu'il sera indiqué explicitement qu'il s'agit de la mise en oeuvre de la méthode de l'invention sur plusieurs fonctions.

Ainsi, le procédé de l'invention permet dans une forme préférée de l'invention de cribler des substances capables de modifier l'activité d'une seule protéine cible exprimée *in vitro* présentant par exemple une activité enzymatique, comme une amylase, une polymérase, une protéase, ou capables de modifier une propriété observable de cette protéine, comme par exemple une activité "DNA-binding", une affinité antigénique, ou encore capables de modifier l'activité ou une propriété d'un variant de cette protéine cible, comme par exemple une amylase thermostable, une polymérase plus processive, une protéase résistante à un anti-protéase, ou une protéine "DNA binding" ayant une plus forte affinité pour l'ADN. Mais le procédé de l'invention permet aussi de cribler des substances capables de modifier l'activité d'un ensemble de protéines exprimées *in vitro.* Dans ce cas c'est l'ensemble de protéines qui constitue la cible des substances à tester. Cet ensemble de protéines exprimées peut correspondre par exemple aux enzymes constituant une voie de synthèse d'un métabolite ou une voie de dégradation d'un composé toxique, mais il peut aussi correspondre à des protéines constituant les sous-unités d'une protéine complexe. Les substances à tester sont donc alors évaluées pour leur capacité à modifier l'activité globale de cet ensemble de protéine, ce qui sous-entend que ces substances peuvent soit modifier l'activité de toutes, d'une parties ou d'une seule des protéines de l'ensemble, car la modification de l'activité d'une ou d'une partie des protéines entraîne la modification de l'activité de l'ensemble. Le procédé de l'invention permet encore de déterminer ou cribler dans une seconde forme de réalisation du procédé de l'invention la ou les protéines exprimées *in vitro* susceptibles de modifier l'activité d'une ou plusieurs substances connues.

La qualité d'une technique de criblage de substances ou protéines capables de modifier l'activité d'une fonction repose principalement sur l'efficacité du test d'activité. Ce test doit être hautement spécifique, sensible, rapide et reproductible. La difficulté des procédés de criblage *in vivo* de telles molécules repose entre autres sur la toxicité et la localisation cellulaire (barrière membranaire) de la protéine cible. La présente invention vise à offrir un procédé d'isolement à haut débit de substances ou protéines susceptibles de modifier l'activité d'une protéine ou plusieurs protéines cibles ou fonctions connues permettant de palier les problèmes ci-dessus.

Ce but est atteint grâce à un procédé de détermination de l'activité *in vitro* d'une substance mettant en oeuvre un test fonctionnel, caractérisé en ce que l'on détecte et/ou mesure une variation de fonction connue correspondant soit à une ou plusieurs protéines produites *in vitro* soit à la substance en présence ou en absence de protéines produites *in vitro.*

Plus particulièrement le procédé de l'invention comprend les étapes suivantes:
a) la préparation d'au moins une molécule d'acide nucléique comprenant le ou les gènes codant pour une ou plusieurs protéines et les éléments de contrôle nécessaires à la transcription et la traduction du ou desdits gènes,
b) la transcription de la ou des molécules d'acide nucléique préparée(s) à l'étape (a),
c) la traduction *in vitro* du ou des transcrit(s) préparé(s) à l'étape (b),
d) la détection et/ou la mesure de la variation d'une fonction connue correspondant à la ou aux protéines produites à l'étape (c) en présence et en absence de ladite substance, ou à la substance en présence et en absence des protéines produites à l'étape (c).

La ou les substances testées dans le cadre du procédé de l'invention sont introduites avant, après et/ou pendant la transcription et/ou la traduction des étapes (b) et/ou (c) et/ou de détection et/ou de mesure de la variation d'au moins une fonction connue de l'étape (d) du procédé de l'invention.

Afin de faciliter l'exposé de l'invention, on désigne à l'étape (a) de la méthode de l'invention, sous le terme de gène, toute séquence d'acides nucléiques permettant l'expression de la ou des protéine(s) correspondant à la ou aux fonction(s) détectée(s). Il peut donc s'agir de séquence d'ADN ou d'ARN.

La préparation d'une ou plusieurs molécules d'acide nucléique de l'étape (a) du procédé de l'invention consiste à placer le ou les gènes codant pour la ou les protéines qui seront produites à l'étape (c), sous le contrôle d'éléments nécessaires à la transcription et à la traduction *in vitro,* c'est à dire sous le contrôle d'un promoteur (en 5') et éventuellement d'un terminateur d'une ARN polymérase (en 3') pour être transcrite in vitro.

Un mode de réalisation particulier de l'invention consiste à utiliser le promoteur et le terminateur de l'ARN polymérase du phage T7 ou SP6 ou Qβ ou λ.

De même, pour que la traduction puisse se faire in vitro, un site de fixation des ribosomes est introduit en amont du gène ou desdits gènes.

Ainsi comme indiqué précédemment, le procédé de l'invention admet deux formes de mise en oeuvre selon que la fonction détectée et/ou mesurée à l'étape (d) correspond soit à la ou aux protéine(s) produite(s) à l'étape (c) soit à la substance. Ce dernier cas étant une forme particulière de la méthode de l'invention.

Dans une première forme de réalisation du procédé de l'invention, le test fonctionnel mis en oeuvre à l'étape (d) correspond à la détection et/ou à la mesure d'une variation de fonction connue de la ou des protéines produites à l'étape (b) en présence et en absence d'une substance. Le procédé de l'invention est donc remarquable en ce qu'il permet le criblage de substances capables de modifier l'activité d'une ou plusieurs protéines cibles (par exemple un ensemble cible de protéines) exprimées *in vitro.*

Le ou les gènes codant pour la ou les protéines correspondant à la fonction connue que l'on souhaite détecter et/ou mesurer à l'étape (d) du procédé de l'invention, sont des gènes connus ou non, synthétiques ou non, placé(s) sous le contrôle des séquences ci-dessus par des techniques classiques connues de l'homme du métier.

Ce ou ces gènes peuvent aussi être présents dans un échantillon contenant des acides nucléiques, comme par exemple un échantillon de sol, de végétal, humain ou animal, d'eau, de culture microbienne, cellulaire ou virale, de biopsie, d'un organisme ou d'un processus. Mais cet échantillon peut également correspondre à des produits de toute méthode d'amplification de l'ADN génomique, de l'ADN synthétique, de l'ARNm, ou tout produit d'acides nucléiques issus de traitements couramment utilisés par l'homme de métier. Il peut s'agir, bien entendu, d'un échantillon biologique brut comme du sang, des tissus, de l'urine, ou tout autre liquide corporel comme le liquide cérébrospinal, synovial, pleural, péricardial, ou préalablement traité pour préparer les acides nucléiques qu'il contient.

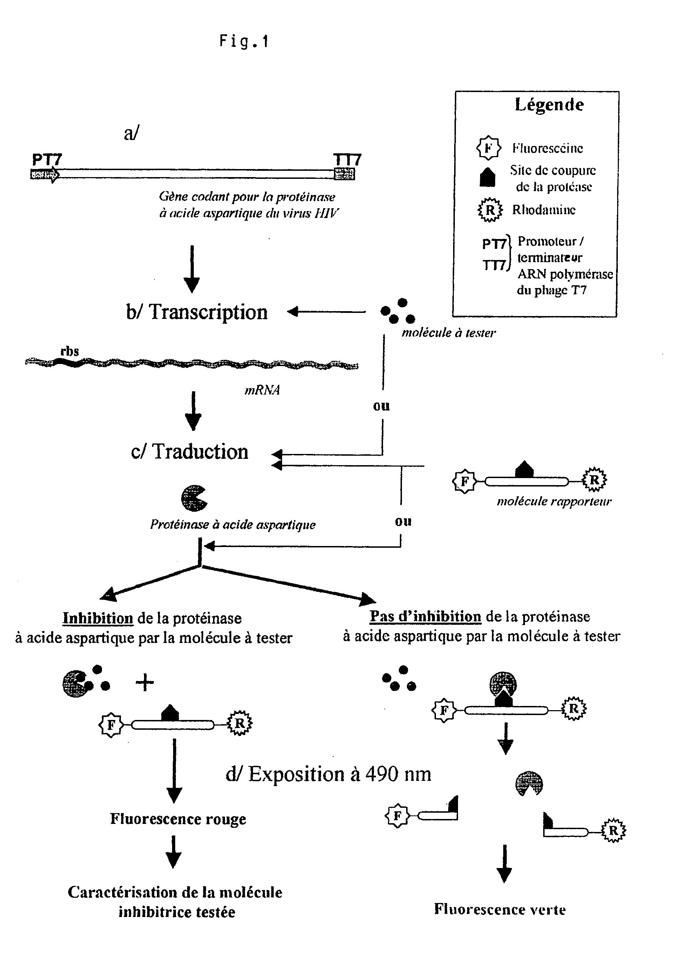

Une forme de mise en oeuvre avantageuse de l'étape (a) du premier mode de réalisation de la méthode de l'invention consiste à préparer la ou les molécules d'acide nucléique par une réaction d'amplification du ou des gènes codant pour la ou lesdites protéines, à partir de l'échantillon d'acides nucléiques. Il peut s'agir d'une amplification par PCR ou par des techniques dérivés de la PCR du type RT-PCR, nested PCR, multiplex PCR, ou des techniques différentes de la PCR du type NASBA, ou rolling circle et autres. Avantageusement cette préparation met en oeuvre un couple d'oligonucléotides ou un couple d'amorces spécifiques de la ou des molécules d'acide nucléique comprenant le ou les gènes codant pour la ou les protéines correspondant à la fonction analysée. Cette préparation par amplification est réalisée à l'aide d'une ou plusieurs paires d'amorces, chacune constituée pour l'exemple de la PCR (figure 1) et de la NASBA :
- pour l'amorce sens, de la séquence s'hybridant en amont d'une ou plusieurs molécules d'acide nucléique comprenant le ou les gènes codant pour la ou lesdites protéines, et d'un promoteur d'ARN polymérase et éventuellement un site de fixation des ribosomes, et
- pour l'amorce antisens, de la séquence s'hybridant en aval d'une ou plusieurs molécules d'acide nucléique comprenant le ou les gènes codant pour la ou lesdites protéines, et éventuellement d'un terminateur d'ARN polymérase.

L'étape (a) peut être réalisée par toute autre technique appropriée. En effet, la préparation de la molécule d'acide nucléique de l'étape (a) peut être réalisée par toute autre méthode connue de l'homme du Métier comme une coupure de restriction permettant de récupérer le ou les gènes d'intérêt suivie d'une ligation orientée avec les éléments de contrôle nécessaires à la transcription et à la traduction in vitro indiqués précédemment .

Comme indiqué précédemment, dans une seconde forme, particulière, de réalisation du procédé de l'invention, le test fonctionnel mis en oeuvre à l'étape (d) correspond à la détection et/ou à la mesure d'une variation de fonction connue de la substance. Cette forme de réalisation, aussi désignée génomique fonctionnelle vise à mesurer la variation d'une fonction connue de la substance en présence d'une ou plusieurs protéines exprimées suivant les étapes (b) à (c) du procédé de l'invention.

Dans ce mode de réalisation, l'étape (a) consiste (i) à préparer à partir d'un échantillon contenant des acides nucléiques plusieurs molécules d'acide nucléique chacune comprenant un fragment d'acide nucléique provenant dudit échantillon, associé avec une molécule vectrice, (ii) à isoler chaque molécule d'acide nucléique constitué d'un fragment d'acide nucléique et d'une molécule vectrice.

Les fragments d'acide nucléique ont de préférence une taille de 1 à plusieurs dizaines de kb, préférentiellement de 1 à 40 kb et avantageusement de 1 à 10 kb lorsque l'échantillon est d'origine procaryote. Ces fragments peuvent porter un opéron partiel ou entier.

Les fragments d'acide nucléique ont de préférence une taille de l'ordre de plusieurs dizaines à plusieurs centaines de kilobases dans le cas d'un organisme eucaryote. Dans le cas particulier où l'on traite des cDNAs à l'étape (a), les fragments auront préférentiellement une taille de 1 à 5 kb dans le cas d'un organisme eucaryote.

La molécule vectrice est composée d'une ou plusieurs séquences polynucléotidiques comportant au moins un promoteur de transcription pour l'étape (b) et éventuellement un élément facilitant l'isolement du fragment d'acide nucléique. Avantageusement cette substance peut être une ou plusieurs molécules de streptavidine ou de biotine, de groupement polypyrol, d'anticorps, une séquence polynucléotidique simple ou double brin, un vecteur d'ADN plasmidique ne comportant préférentiellement pas de séquences permettant l'expression *in vivo* du fragment associé, ou tout autre composé permettant l'isolement du fragment d'acide nucléique.

De préférence, la molécule vectrice est composée de deux séquences polynucléotidiques comportant chacune au moins un promoteur de transcription, chacune de ces séquences étant associée à une extrémité d'un des fragments d'acide nucléique. Le ou les promoteurs de transcription portés par la molécule vectrice sont préférentiellement de type fort.

La molécule vectrice associée à chaque fragment de l'étape (b) est avantageusement un vecteur plasmidique ne permettant préférentiellement pas l'expression dudit fragment *in* vivo .

Dans le second mode de réalisation de l'invention, l'échantillon biologique à partir duquel sont préparées les molécules d'acide nucléique de l'étape (a) peut provenir d'un ou plusieurs organismes procaryotes ou cellules d'eucaryotes, ou encore de virus, identiques ou différents, mais il peut aussi être constitué d'une séquence ou d'une banque d'acides nucléiques synthétiques ou encore est constitué d'organismes et/ou d'acides nucléiques inconnus. Il peut s'agir d'une banque d'ADN eucaryote et alors la réaction de transcription de l'étape (b) est complétée par une réaction d'épissage et de maturation *in vitro* des ARNm en utilisant un extrait nucléaire.

Comme indiqué ci-dessus, selon une forme de réalisation particulière du procédé de l'invention, la molécule vectrice associée aux fragments d'acide nucléique est un vecteur plasmidique. Dans ce cas, chaque fragment est inséré dans un vecteur au niveau d'un site de clonage ou d'une cassette de restriction. Ce vecteur plasmidique est caractérisé en ce qu'il comprend un promoteur d'ARN polymérase d'un côté du site de clonage et éventuellement d'un terminateur d'ARN polymérase de l'autre côté. Il est aussi possible de concevoir un vecteur comprenant un site de clonage encadré par deux promoteurs d'ARN polymérase identiques ou différents et éventuellement flanqués de part et d'autre du ou des terminateurs d'ARN polymérase correspondants. Ces promoteurs et éventuellement terminateurs ont préférentiellement pour caractéristique de ne pas fonctionner dans le micro-organisme pouvant être utilisé pour la séparation des vecteurs recombinants à l'étape.

Dans le cas où le vecteur ne possède pas de promoteur(s) et/ou d'éventuel terminateur(s) d'ARN polymérase, ou dans le cas ou les promoteur(s) et éventuels terminateur(s) d'ARN polymérase ne sont pas adéquats pour réaliser l'étape (b), ces promoteur(s) et éventuels terminateur(s) peuvent être insérés par tout moyen approprié. Une mise en oeuvre avantageuse de cette insertion consiste à réaliser une PCR avec un jeu d'amorces portant les séquences des promoteur(s) et terminateur(s). Selon une forme de mise en oeuvre particulière du procédé de l'invention, les promoteur(s) et éventuels terminateur(s) sont de type forts comme par exemple ceux de la T7 ou SP6 ou Qβ ARN polymérase.

Dans le cas, où la molécule vectrice est un vecteur plasmidique, l'isolement du vecteur recombinant peut être réalisé par transformation de cellules hôtes par l'ensemble des vecteurs recombinants de façon à créer une banque de clones, puis en ce qu'on réalise une extraction du vecteur ou d'une partie du vecteur recombinant contenu par chaque clone de la banque par tout moyen approprié.

L'extraction du vecteur recombinant ou d'une partie de vecteur recombinant flanquée de promoteur(s) et d'éventuel(s) terminateur(s) d'ARN polymérase peut être réalisée par toute méthode connue de l'homme du métier, comme par mini préparation et éventuellement digestion ou par PCR. Une alternative avantageuse consiste à réaliser cette PCR avec des oligonucléotides protégés en 5' des attaques nucléasiques, notamment des nucléases contenues dans le milieu de traduction, par des groupements phosphorothioates.

Comme indiqué précédemment, l'isolement des molécules d'acide nucléique peut être réalisé par tout moyen physique, mécanique ou chimique comme par exemple une simple dilution extrême de l'ensemble des fragments associés à la molécule vectrice. Mais l'isolement peut aussi avantageusement être réalisé en utilisant les propriétés d'une substance spécifique incluse dans la molécule vectrice, comme une molécule d'anticorps, et l'isolement du fragment est réalisé en utilisant l'affinité anticorps-antigène ; ou une biotine, et l'isolement est réalisé en utilisant l'affinité biotine-streptavidine, etc... .

Le procédé de criblage selon l'invention accepte plusieurs modes de mises en oeuvre notamment en fonction du type d'activité de la ou des protéines cibles. En effet, le ou les gènes cibles préparés à l'étape (a) peuvent coder pour une ou plusieurs protéines cibles ou fonctions impliquées dans divers types de processus biologiques. Ce(s) gène(s) peuvent provenir de plusieurs organismes procaryotes ou de cellules eucaryotes ou encore de virus. Comme indiqué précédemment, un processus peut être par exemple une maladie infectieuse, un mécanisme de résistance bactérien ou viral, une chaîne métabolique ou encore une processus impliqué dans un procédé industriel de l'agroalimentaire, de traitement du papier, de préparation de détergents, de fabrication de textile etc....

L'étape (b) de transcription et la phase de traduction de l'étape (c) peuvent être simultanées, ce qui signifie que la phase de traduction de l'étape (c) est réalisée simultanément avec la transcription de l'étape (b), ou décomposées en deux étapes distinctes, (b) de transcription et (c) de traduction.

Le découplage des étapes (b) et (c) permet d'optimiser les rendements de chaque étape, et ainsi de produire des quantités plus importantes de protéines, ce qui trouve toute son utilité dans la cas détection d'enzymes de faible activité spécifique.

Ce découplage permet aussi de normaliser la formation des produits à l'étape (c) et de pouvoir comparer ultérieurement les différentes fonctions exprimées.

Le découplage entre la transcription de l'étape (b) et la traduction de l'étape (c) permet également d'éviter les problèmes de dégradation de la matrice ADN par les nucléases si celle-ci a été préparée par PCR. En effet, les composants de la réaction de transcription sont moins contaminés par des nucléases, contrairement aux extraits de traduction.

Le découplage permet en outre l'emploi d'extraits de traduction différents selon l'origine de l'ADN criblé. En effet, la phase de traduction du transcrit à l'étape (c) est avantageusement réalisée avec un extrait de traduction de la même origine ou d'une origine proche de celle de l'échantillon biologique sur lequel est pratiqué le procédé de l'invention. Ainsi, on optimise l'adéquation entre l'origine des signaux de traduction des transcrits et l'extrait de traduction pour une efficacité de traduction optimale. On peut citer à titre d'exemple, l'utilisation d'un extrait de traduction d'un organisme extrêmophile si la préparation à l'étape (a) met en oeuvre un échantillon d'acide nucléique provenant du même organisme ou d'un autre organisme extrêmophile (thermophiles, halophiles, acidophiles, etc...) ou encore un extrait de traduction de cellules eucaryotes si la préparation à l'étape (a) met en oeuvre un échantillon d'acide nucléique eucaryote. Ces extraits respectifs sont susceptibles d'améliorer l'efficacité du procédé. Ces extraits sont choisis pour leur capacité à traduire les transcrits à l'étape (c).

Le procédé de l'invention est remarquable en ce qu'il met en oeuvre une adéquation entre la ponctuation d'expression des transcrits de l'étape (b) et les extraits de traduction utilisés. Ces extraits sont aussi caractérisés en ce que soit ils ne contiennent pas la fonction recherchée, soit ils la contiennent mais qu'elle n'est pas détectable dans les conditions de test réalisées pour détecter la fonction recherchée. Il s'agit par exemple de l'utilisation d'un extrait de traduction contenant une activité beta-galactosidase mésophile permettant de traduire un ARNm d'une beta-galactosidase thermophile et de la détection de l'activité de cette dernière à haute température, ce qui élimine l'activité beta-galactosidase mésophile.

En fonction de l'origine génétique des gènes obtenus à l'étape (a), par exemple ADN de microorganismes Gram positifs, ou négatifs, d'eucaryotes, de virus etc..., et de la fonction testée, différents extraits de traduction peuvent donc être utilisés.

Une mise en oeuvre particulière du procédé de l'invention consiste à utiliser à l'étape (c) un extrait de traduction qui soit en fait un mélange de plusieurs extraits de traduction. Il peut s'agir par exemple d'extrait de traduction de *E*. *coli* surexprimant une protéine chaperon A mélangé avec un extrait de traduction de *E*. *coli* surexprimant une protéine chaperon B. Tout type de mélange est envisageable du moment qu'il correspond aux caractéristiques décrites ci-dessus. De la même manière, il est possible d'utiliser un extrait de traduction dans lequel est rajouté un ou plusieurs tRNAs spécifiques d'un ou de plusieurs codons. Les extraits de traduction ainsi obtenus permettent alors de traduire des mRNA comportant ces codons spécifiques, comme par exemple la traduction d'un mRNA contenant un codon ambre en rajoutant dans l'extrait de traduction un ou des tRNAs suppresseurs.

Le traitement de l'étape (c) avec un extrait de traduction peut aussi être réalisé avec un extrait universel de traduction quelque soit l'origine de l'échantillon comme par exemple un extrait *d'E. coli* et/ou tout(s) autre(s) extrait(s) cellulaire(s) supplémentés ou non par des molécules intéressantes comme celles, par exemple, indiquées précédemment (tRNA, chaperon...).

Il est également possible d'ajouter à l'extrait de traduction de l'étape (c) une ou plusieurs substances favorisant un repliement ou une maturation plus efficace des protéines exprimées, comme par exemple des chaperons, des détergents, des sulfobétaïnes, des extraits membranaires, etc....

La détection et/ou la mesure de la variation d'au moins une fonction connue correspondant à la ou aux protéines produites à l'étape (c) ou à la substance est réalisée par tout test fonctionnel connu de l'homme du métier comme défini en introduction. L'étape (d) peut ainsi consister à détecter et/ou mesurer plusieurs variation de fonctions correspondant à une ou plusieurs des protéines produites à l'étape (d) ou correspondant à une ou plusieurs substances.

La(es) variation de fonction(s) sont détectée(s) ou dosée(s) de manière directe ou indirecte par un ou plusieur(s) test(s) fonctionnel(s) de la ou des protéine(s) produite(s) à l'étape (c) ou de la ou des substance(s) à cribler. La(s) fonction(s) est(sont) lue(s) par exemple en continu par exemple par fluorométrie ou par colorométrie ou par viscosimétrie ou par spectrométrie de masse...

La détection et/ou la mesure de la variation de fonction correspondant à la ou aux protéine(s) produite(s) à l'étape (c) ou à la ou aux substance(s) est avantageusement réalisée à l'étape (d) par un test fonctionnel mettant en oeuvre la présence à l'une des étapes (a), (b), (c) ou (d) d'une ou plusieurs molécule(s) rapporteur permettant de détecter et/ou de mesurer l'activité de la ou des protéine(s) produite(s) à l'étape (c) ou de la ou des substance(s) à cribler correspondant à la fonction analysée à l'étape (d).

Le procédé de l'invention tant dans son premier que dans son second mode de réalisation présente les avantages suivants :
- Le fait de travailler directement sur l'activité de la ou des protéines produites à l'étape (c) ou sur l'activité de la substance permet une bonne spécificité du test de criblage.
- La sensibilité du test s'explique par le coefficient multiplicateur des étapes enzymatiques (b) et (c), correspondant respectivement à la transcription et à la traduction, et éventuellement au test fonctionnel mettant en oeuvre une enzyme.
- Le procédé de l'invention est rapide car entièrement automatisable. Par exemple une plaque de 384 puits peut être traitée en 2 à 3 heures.
- Enfin la reproductibilité est facilitée par l'absence de stockage de la ou des protéines produites à l'étape (c). De plus, la protéine ou les protéines sont exprimées *in vitro,* indépendamment d'un contexte cellulaire complexe et pas toujours contrôlable (diffusion membranaire, localisation cellulaire, toxicité cellulaire, physiologie cellulaire, problèmes d'induction ou de répression, etc...).

Le procédé de l'invention est donc remarquable car outre la réalisation d'un criblage de substances sur une ou plusieurs fonctions, il permet aussi de :
- de développer de nouveaux test fonctionnels
- de faire de la génomique fonctionnelle.

Le procédé de l'invention permet en effet de développer de nouveaux tests fonctionnels. Nous avons assisté ces dernières années à un essor des tests de criblage à haut débit ou "high throuput screening (HTS)". Ces criblages, pour être optimaux, nécessitent la mise en oeuvre de tests fonctionnels sensibles, or on ne dispose pas toujours de ces tests fonctionnels. Le procédé de l'invention permet d'identifier un ou plusieurs test(s) fonctionnel(s) permettant d'améliorer la révélation d'une ou plusieurs fonction(s) d'un processus ou d'un organisme, et ainsi d'améliorer la mesure de la variation de cette ou de ces fonction(s) en présence ou en absence d'une ou plusieurs substance(s).

Ainsi il est possible de développer de nouveaux tests fonctionnels notamment de la façon suivante :
a) la préparation d'au moins une molécule d'acide nucléique comprenant le ou les gènes codant pour une ou plusieurs protéines et les éléments de contrôle nécessaires à la transcription et à la traduction du ou desdits gènes.
b) La transcription de la ou des molécules d'acide nucléique préparées à l'étape (a).
c) La traduction in vitro du ou des transcrit(s) préparé(s) à l'étape (b).
d) La détection et/ou la mesure de la variation d'une fonction connue correspondant aux protéines produites à l'étape (c) en présence et en absence d'une ou plusieurs molécules rapporteur(s).

Ainsi dans le cadre de cette application la substance correspond à la molécule rapporteur capable de révéler une fonction.

Le procédé de l'invention dans son premier et second mode de réalisation présente un réel avantage pour la génomique fonctionnelle.

Le procédé de l'invention s'applique tout particulièrement à l'après séquençage génomique. Le projet de décodage du génome humain a donné naissance à une nouvelle science la génomique, maintenant présente au coeur de l'entreprise thérapeutique. La génomique permet d'identifier et de décrire les gènes qui dictent la fabrication et l'agencement de toutes les molécules d'un organisme. Ces gènes codant pour des fonctions d'organismes ou de processus peuvent être exprimés par le procédé de l'invention, qui permet de confirmer la fonction codée par un cadre de lecture repéré par bio informatique, de mettre en évidence une ou plusieurs substances pharmaceutiques capables de modifier la dite fonction codée par une ou plusieurs protéines exprimées par le procédé de l'invention ou qui permettra d'identifier les cibles d'une substance correspondant à une ou plusieurs fonctions d'un organisme ou d'un processus exprimé à l'étape (c).

En 100 ans d'existence, l'industrie pharmaceutique a identifié plusieurs centaines de sites récepteurs. Avec la génomique fonctionnelle, on pourra accéder à d'autres sites récepteurs existants, autant de fonctions cibles potentielles pour de futurs substances qu'il reste à concevoir.

A titre d'exemple d'une telle application, on peut citer le repérage par bioinformatique d'un ORF codant éventuellement pour une cible, confirmer cette cible par le procédé de l'invention et lui trouver des substances qui peuvent faire varier son activité.

Un autre exemple de la génomique fonctionnelle consiste à identifier au niveau génétique un gène correspondant à une protéine dont l'activité est modifiée par une plusieurs substances. Dans le cas où l'on connaît l'effet d'une substance pharmaceutique sur un organisme ou un processus, cette application du procédé de l'invention présente un intérêt tout particulier. Les gènes correspondant aux protéines codées par cet organisme ou ce processus seront alors préparés selon une forme particulière de mise en oeuvre de l'étape (a) décrite précédemment, de manière à isoler lesdits gènes, et à les exprimer aux étapes (b) et (c) du procédé de l'invention. Un test fonctionnel à l'étape (d) permet de mesurer l'activité de la substance en présence et en absence de chacune des protéines exprimées à l'étape (c). La mesure à l'étape (d) d'une variation d'activité de la substance en présence et en absence de protéines exprimées à l'étape (c) permet alors d'identifier la ou les protéine(s) dont l'activité est modifiée par ladite substance au sein de l'organisme ou du processus.

Cette mise en oeuvre de l'invention présente l'énorme avantage de pouvoir remonter directement au gène à partir de la ou des protéines exprimées à l'étape (c) et isolées à l'étape (a). On peut ainsi identifier la cible fonctionnel au niveau génétique d'une ou plusieurs substances.

A chaque fois qu'un test fonctionnel peut être mis en oeuvre (test enzymatique, test de binding, etc...), le procédé de l'invention permet de soumettre à haut débit l'ensemble des protéines exprimables d'un organisme considéré à un test fonctionnel automatisé, et de remonter en quelques heures aux gènes correspondant à la fonction recherchée.

La description qui suit se rapporte de préférence à des exemples de réalisation du premier mode de mise en oeuvre du procédé de l'invention dans lequel la ou les protéines correspondant à la ou aux fonctions détectées et/ou mesurées à l'étape (d) sont désignées protéine(s) cible(s) et le ou les gènes codant correspondant est(sont) désignés gène(s) cible(s). Ainsi, le procédé de l'invention permettant de cribler des substances capables de modifier l'activité d'une protéine cible ou plusieurs protéines cibles exprimées *in vitro* comprend les étapes suivantes:
a) la préparation d'au moins une molécule d'acide nucléique comprenant le ou les gènes codant pour la ou les protéines cibles et les éléments de contrôle nécessaire à la transcription et à la traduction du ou desdits gènes cibles,
b) la transcription *in vitro* de la ou des molécules d'acide nucléique préparée à l'étape (a),
c) la traduction *in vitro* des transcrits de l'étape (b),
d) la détection et/ou la mesure de la variation d'au moins une fonction connue correspondant à la ou aux protéines cibles produites à l'étape (c) en présence et en absence de la substance à cribler.

Comme indiqué précédemment le procédé de l'invention permet de détecter et/ou mesurer à l'étape (d) la variation d'une ou plusieurs fonctions correspondant à une ou plusieurs protéines cibles produites à l'étape (c). Il peut s'agir par exemple des protéines exprimées par un opéron. En conséquence, le procédé de l'invention accepte plusieurs formes de réalisation.

Lorsqu'il concerne le criblage de substances susceptibles de modifier une fonction correspondant à l'activité d'une protéine cible, le procédé de l'invention comprend les étapes suivantes :
a) la préparation d'une molécule d'acide nucléique comprenant le gène codant pour ladite protéine, en 5' dudit gène un promoteur d'ARN polymérase et un site de fixation des ribosomes, et éventuellement un terminateur d'ARN polymérase en 3' dudit gène,
b) la transcription *in vitro* de la molécule d'acide nucléique préparée à l'étape (a),
c) la traduction *in vitro* des transcrits de l'étape (b),
d) la détection et/ou la mesure de la variation d'au moins une fonction connue correspondant à la ou aux protéines cibles produites à l'étape (c) en présence et en absence de la substance à cribler.

Mais le procédé de l'invention peut aussi être appliqué au criblage de substances capables de modifier la fonction correspondant à un ensemble de protéines cibles. Les gènes codant pour ces protéines peuvent être situés sur le même fragment d'ADN comme dans le cas d'un opéron, ou à différents endroits de l'ADN génomique comme dans le cas de certaines voies métaboliques.

Lorsque le procédé de l'invention concerne le criblage d'une substance capable de modifier l'activité d'un ensembles de protéines, l'étape (a) du procédé admet les deux formes de mise en oeuvre suivantes :
i) Soit, les gènes sont regroupés sous la forme d'un opéron, et alors l'étape (a) consiste à préparer une molécule d'acide nucléique comprenant les gènes (l'opéron) codant pour les protéines, en 5' de l'ensemble desdits gènes (de l'opéron) un promoteur d'ARN polymérase, éventuellement en 3' de l'ensemble desdits gènes (de l'opéron) un terminateur d'ARN polymérase, et pour chacun desdits gènes son site de fixation des ribosomes naturel.
ii) Soit, lesdits gènes sont séparés et alors l'étape (a) consiste à préparer une ou plusieurs molécules d'acide nucléique comprenant les gènes codant pour les protéines, en 5' de chacun desdits gènes un promoteur d'ARN polymérase et un site de fixation des ribosomes, et éventuellement en 3' de chacun desdits gènes un terminateur d'ARN polymérase.

Dans la première forme de mise en oeuvre (i) ci-dessus, le site de fixation des ribosomes de chacun des gènes est son site de fixation des ribosomes naturel, et l'on préfère alors utiliser à l'étape (c) un extrait de traduction préparé à partir de l'organisme dont provient le ou les gènes cibles ou d'un organisme phylogénétiquement proche.

Dans la seconde forme de mise en oeuvre (ii) ci-dessus, le site de fixation des ribosomes peut être le site naturel de chacun des gènes ou un autre site de fixation des ribosomes plus adapté à l'étape (c) de traduction.

Une variante de la première (i) et de la deuxième forme (ii) de mise en oeuvre du procédé ci-dessus, consiste à réaliser en parallèle ou simultanément, le procédé de l'invention décrit précédemment avec une seule protéine, chaque étape (a) étant réalisée avec chacun des gènes. Une alternative à la réalisation en parallèle ou simultanée de procédés de l'invention, consiste à réaliser séparément pour chacun des gènes les étapes (a), (b) et (c), puis, pour le criblage final impliquant chacune des protéines, à rassembler les produits des étapes (c) pour réaliser l'étape (d).

De même, lorsque le procédé concerne le criblage de substances capables de modifier l'activité d'un ensemble de protéines dont les gènes sont physiquement séparés sur le génome d'un organisme, le procédé de l'invention consiste à réaliser en parallèle ou simultanément, le procédé de l'invention décrit précédemment avec une seule protéine, chaque étape (a) étant réalisée avec chacun des gènes. Une alternative à la réalisation en parallèle ou simultanée de procédés de l'invention, consiste à réaliser séparément pour chacun des gènes, les étapes (a), (b) et (c) puis, pour le criblage final impliquant chacune des protéines, à rassembler les produits des étapes (c) pour réaliser l'étape (d).

Comme indiqué précédemment, le procédé de l'invention permet de cribler des substances capables de modifier l'activité d'un ensemble de protéines cibles. Le procédé de l'invention peut donc être appliqué au criblage de substances capables de modifier l'activité des variants d'une protéine ou des variants d'un ensemble de protéines. Le ou les gènes correspondants à ce ou à ces variants peuvent être contenus par exemple dans un même échantillon d'acides nucléiques de départ. A titre d'exemple de cette application du procédé de l'invention, on peut citer les différents mutants du gène de la protéase du VIH contenus dans un échantillon d'un patient infecté par ce virus. La mise en oeuvre du procédé de l'invention consiste alors à réaliser chaque étape (a) avec chacun des mutants dudit gène de façon à exprimer chacun de ceux-ci séparément. La séparation des mutants contenus dans l'échantillon peut être réalisée par clonage, dilution extrême, ou par toute autre méthode connue de l'homme du Métier. Cette application du procédé de l'invention consiste donc à cribler toutes les substances à tester non pas sur une protéine cible ou sur un ensemble de protéines cibles mais sur tous les variants existant de celle(s)-ci, dont les gènes correspondant sont contenus dans un seul échantillon d'acides nucléiques. Cette application du procédé de l'invention permet donc de déterminer le spectre d'action des substances à tester.

L'invention se rapporte donc aussi à l'application du procédé de criblage de substances capables de modifier les différents variants d'une protéine cible ou d'un ensemble de protéines cibles. En effet, comme indiqué ci-dessus sur la protéase du VIH, l'échantillon d'un patient infecté par ce virus peut contenir plusieurs variants du virus chacun exprimant une protéase différente. Il est donc intéressant d'effectuer un criblage sur chacune des différentes protéases afin de déterminer celles qui sont inhibées par les antiprotéases connues ou celles qui peuvent l'être par de nouvelles substances à tester.

Sur la base de cet exemple du virus HIV, l'invention permet l'analyse *in vitro* des différents variants d'une protéine cible ou d'un ensemble de protéines cibles. Ce but est atteint selon l'invention, en amplifiant éventuellement les différents mutants, par exemple par culture cellulaire ou par amplification moléculaire, puis en isolant chaque gène mutant, par exemple par clonage ou par dilution extrême, et en exprimant chacun de ces gènes conformément aux étapes (a), (b) et (c) du procédé de l'invention, et enfin en criblant les substances capables de modifier l'activité de la ou des protéines produites lors de l'étape (d). Le procédé de criblage consiste par exemple dans le cas des protéases du VIH à réaliser un test d'inhibition avec les différents inhibiteurs ou les différentes substances à tester sur chaque protéase individuellement. L'activité de chacune des protéases exprimées conformément au procédé de l'invention peut alors être caractérisée de façon à révéler la représentation des différents variants du virus qui infectent un patient. Le résultat du procédé de criblage selon l'invention permet alors d'adapter la thérapie du patient.

Comme on l'a dit précédemment, le test fonctionnel peut mettre en oeuvre une ou plusieurs molécules rapporteurs. Dans un premier mode particulier de réalisation du procédé de l'invention, la molécule rapporteur doit être présente à l'étape (c) ou (d) pour révéler l'activité éventuelle de la protéine cible en fonction du rôle de la substance testée lors du criblage.

Mais la molécule rapporteur peut aussi être présente dans le mélange réactionnel dès l'une des étapes (a) à (b), soit sous sa forme finale de molécule rapporteur, soit, selon une forme particulière de réalisation de l'invention, sous la forme d'une molécule d'acide nucléique (ADN, ou ARN) correspondant au gène codant pour ladite molécule rapporteur, et alors désigné ci-après gène rapporteur. Dans ce mode de réalisation particulier, la molécule rapporteur sera avantageusement produite lors de l'étape (c) conjointement avec la ou les protéines cibles.

Ainsi, selon une forme particulière de mise en oeuvre du procédé de criblage de l'invention, la molécule rapporteur est une protéine qui est produite lors de l'étape (c) conjointement avec la ou les protéines cibles. Avantageusement, dans cette forme de réalisation du procédé de l'invention, le gène codant pour la molécule rapporteur est placé sous le contrôle de séquences de régulation de la transcription et de la traduction similaires à celle du ou des gènes codant pour la ou les protéines cibles, de façon à ce que le gène rapporteur soit coexprimé avec le ou les gènes cibles.

A titre d'exemple, le gène rapporteur peut être le gène de la protéine GFP (Green Fluorescent Protein) ou celui de la beta-lactamase (TEM-1). Dans le cas de la GFP, c'est l'émission de fluorescence qui est évaluée. Il est alors possible de réaliser un test tel que la GFP ne fluorèsce que si l'activité des produits du ou des gènes cibles est modifiée par la ou les molécule(s) testée(s). Dans le cas de la beta-lactamase, c'est l'activité de cette enzyme qui est évaluée en incubant une fraction de la réaction de traduction dans un tampon contenant de la nitrocéphine. La nitrocéphine est une beta-lactamine chromogénique qui a la propriété de virer du jaune au rouge lorsqu'elle est hydrolysée par une beta-lactamase active. Un test jaune indique par exemple que la ou les molécule(s) testée(s) est (sont) capable(s) de modifier l'activité du produit du gène cible.

Tout autre gène rapporteur peut être envisagé dans le procédé de l'invention, comme ceux de la beta-galactosidase, la luciférase, la peroxydase ou une microperoxydase, etc... .Il convient de noter que le gène rapporteur de la GFP présente l'avantage de produire une protéine, dont l'activité est instantanément mesurable, ce qui permet un gain de temps supplémentaire. Mais, un gène rapporteur codant pour une protéine ayant une activité enzymatique du type beta-lactamase présente une grande sensibilité due au coefficient enzymatique multiplicateur.

Une forme particulière de mise en oeuvre du procédé de l'invention avec un gène rapporteur, consiste à exprimer *in vitro* un gène rapporteur au sein duquel un gène ou plusieurs gènes codant pour la ou les protéines ont été insérés, l'ensemble de ces gènes étant sous le contrôle des mêmes séquences régulatrices de la transcription et de la traduction.

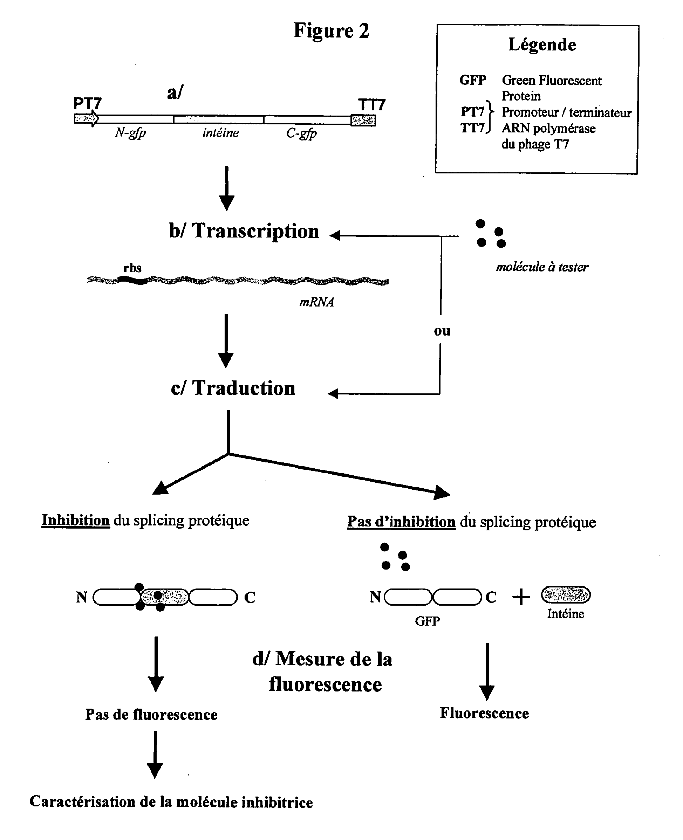

Un exemple de molécule d'acide nucléique préparée à l'étape (a) correspondant à cette forme de mise en oeuvre du procédé de l'invention est représentée à la figure 2 en annexe. Ce cas particulier, où le gène cible et le gène rapporteur sont coexprimés, est tout à fait adapté au gène cible correspondant aux intéines comme l'intéine de RecA de *Mycobacterium tuberculosis.*

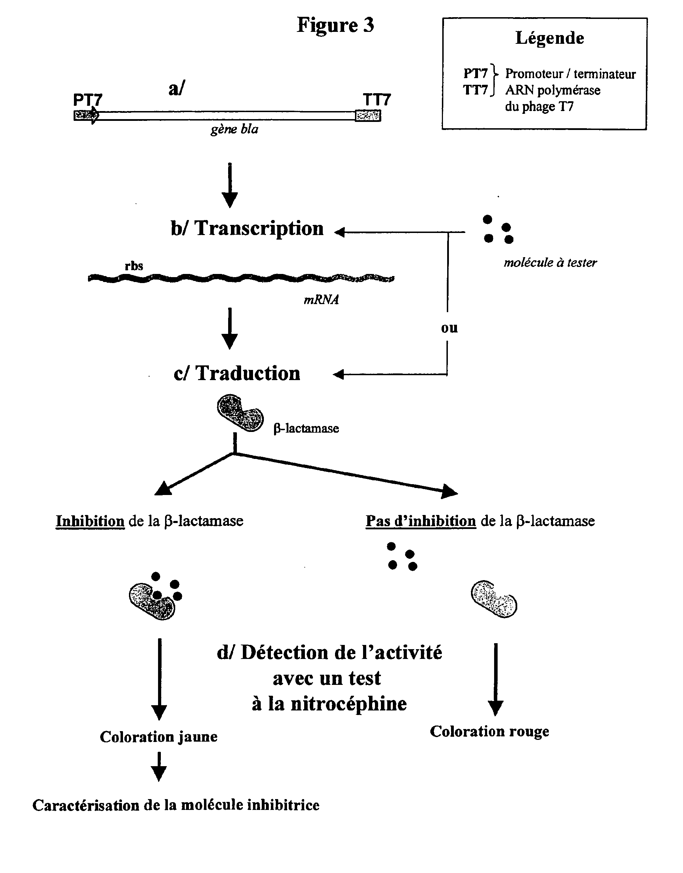

Une autre forme particulière de mise en oeuvre du procédé de l'invention avec un gène rapporteur, consiste à utiliser un gène cible (codant pour une protéine cible ou une fonction) qui peut être lui même un gène rapporteur. Un exemple de molécule d'acide nucléique préparée à l'étape (a) correspondant à cette forme de mise en oeuvre du procédé de l'invention est représentée à la figure 3 en annexe. Ce mode de mise en oeuvre est particulièrement adapté à une protéine cible ayant une activité directement détectable *in vitro,* comme une activité enzymatique, telle que la beta-lactamase. Le gène de la beta-lactamase joue un rôle essentiel dans la résistance aux antibiotiques de type beta-lactamine en hydrolysant ces molécules avant qu'elles n'aient pu agir sur leurs cibles thérapeutiques. Ainsi, une fois que l'enzyme a été mise en présence de la molécules à tester à l'étape (b), (c) ou (d), l'activité de la beta-lactamase peut être évaluée grâce à un test à la nitrocéphine.

Les étapes du procédé de l'invention peuvent être réalisées successivement sans interruption par le même opérateur, avantageusement sur un dispositif automatisé intégrant chacune des étapes, ou peuvent être réalisées de façon discontinues, éventuellement par des opérateurs différents.

Si la substance testée correspond à une séquence polynucléotidique, elle pourra avoir un rôle en tant que tel ou en tant que gène codant pour une protéine. Dans ce dernier cas, cette séquence polynucléotidique possède toutes les séquences nécessaires pour son expression *in vitro* lors de la réaction de transcription et de traduction. De préférence, elle sera exprimée par les mêmes séquences régulatrices que le ou les gènes cibles afin que leur expression soit concomitante et que la molécule ait le temps de modifier l'activité des produits du ou des gènes exprimés après les étapes (b) et (c). Ceci évite de générer des résultats faux positifs ou faux négatifs.

Un des rôles possibles des substances à cribler est de ralentir voir de stopper, *in vitro,* l'activité d'une protéine cible ou d'un ensemble de protéines cibles, qui peuvent être impliquées par exemple dans la progression d'une maladie. Ces molécules seront par exemple issues de bibliothèques présentant un intérêt pharmacologique particulier, issues de travaux de chimie combinatoire tenant compte de la structure d'inhibiteurs puissants préexistants et/ou de la structure du site actif d'une enzyme cible et de ses caractéristiques particulières.

Mais les substances testées dans le cadre du procédé de l'invention peuvent également avoir un effet stimulateur ou activateur sur l'activité du produit d'un gène ou plusieurs gènes cibles ayant un rôle clé dans un processus. De telles molécules peuvent s'avérer utiles en tant que cofacteurs enzymatiques permettant de démultiplier l'activité d'une enzyme dans un phénomène industriel donné (agroalimentaire ou autre) et ainsi de gagner en rendement.

A chaque fois qu'une substance testée s'avère efficace pour modifier l'activité de la protéine cible, il est avantageux de vérifier, par toute méthode appropriée, que cette substance n'inhibe pas une des étapes du procédé. En outre, après avoir identifié une substance capable de modifier l'activité d'une protéine cible, cette protéine peut être testée selon le procédé de l'invention sur des protéines ayant une activité similaire à celle de la ou des protéines cibles.

Le procédé de l'invention peut être avantageusement mis en oeuvre sur tout type de support et est donc facilement automatisable. On entend par support par exemple des puits de plaques de microtitration et également des biopuces. Celles-ci peuvent contenir plusieurs dizaines à plusieurs milliers d'emplacements. Ainsi sur un seul support des dizaines à des milliers de substances pourront être testées pour leur capacité à modifier l'activité codée par le ou les gènes cibles d'une ou plusieurs molécules d'acide nucléique préparée à l'étape (a).

Une mise en oeuvre particulière du procédé de l'invention consiste à charger les puits de ces plaques avec un mélange de réaction de transcription et de traduction concentrée couplée (composants nécessaires à la transcription et à la traduction) contenant une ou plusieurs molécules d'acide nucléique préparée à l'étape (a) et la molécule rapporteur, puis à les congeler pour éviter l'initiation précoce de la réaction de transcription/traduction. Les plaques congelées sont placées sur un automate, qui dépose dans chaque puits un volume de la molécule à tester permettant de diluer le mélange de transcription et de traduction jusqu'à ce qu'il soit concentré 1 fois. Une homogénéisation peut être envisagée par l'intermédiaire de l'automate, mais les volumes réactionnels devraient être suffisamment faibles pour que la diffusion assure cette homogénéisation.

La mesure de l'activité d'une ou plusieurs protéines cibles ou de la molécule rapporteur de l'étape (d), et donc de l'effet de la substance testée sur une protéine cible ou un ensemble de protéines cibles, peut également être avantageusement automatisée. La modification éventuelle de l'activité de la protéine cible est lue directement sur le support dans un lecteur de fluorimétrie (si le rapporteur est par exemple la GFP) ou de colorimétrie (si le rapporteur est par exemple la beta-lactamase). Les lectures automatisées seront adaptées à la révélation du rapporteur. Il est ainsi possible de réaliser une lecture en continue de l'activité du rapporteur.

L'automatisation présente des avantages en terme de potentiel de criblage. Le procédé de l'invention peut être envisagé dans un concept de micro-usinage de manière à manipuler sur un support unique des milliers de nanovolumes réactionnels. Ceci représente un avantage certain en terme de coût mais également en potentiel de criblage à haut débit. Le procédé de l'invention mis en oeuvre sur biopuce permet de cribler des collections de substances par exemple issues de travaux de chimie combinatoire présentant par exemple des propriétés antifongiques ou antibactériennes ou antivirales ou anticancéreuses ou des collections de substances dirigées contre des maladies comme celles de Parkinson ou d'Alzheimer. Le procédé de l'invention appliqué à haut débit permet d'identifier la substance-clef la mieux adaptée à une fonction-serrure donnée en un minimum de temps. Il permet donc de tester rapidement et systématiquement toutes les combinaisons possibles d'un jeu de substances données.

En conséquence, l'invention concerne un dispositif comprenant un agencement d'un ou plusieurs supports, de robots et d'un lecteur desdits supports pour la réalisation des étapes du procédé de l'invention.

L'invention a enfin aussi pour objet un kit de criblage de substances capables de modifier l'activité d'une ou plusieurs fonctions *in vitro* conformément à l'une des formes de mise en oeuvre du procédé de l'invention.

Dans une première forme de réalisation un tel kit comprend les moyens de révélation de la fonction, une ARN polymérase, des séquences nucléotidiques pour la préparation des molécules d'acides nucléiques comprenant le ou les gènes permettant l'expression de la ou des protéines correspondant à la fonction détectée et/ou quantifiée, les quatre nucléotides triphosphates, les mélanges nécessaires à ladite préparation, à la transcription et à la traduction, éventuellement des substances, éventuellement des témoins.

Dans une seconde forme de réalisation un kit selon l'invention comprend :
- éventuellement des substances nécessaires à la préparation des molécules d'acides nucléiques comprenant le ou les gènes permettant l'expression de la ou des protéines correspondant à la fonction détectée et/ou quantifiée,
- tout support comme plaque de microtitration ou puce contenant : les moyens de révélation de la fonction, une ARN polymérase, les quatre nucléotides triphosphates, les mélanges de transcription et de traduction, éventuellement des témoins.

Les kits et les supports peuvent être envisagés pour la détection et/ou la mesure d'une variation simultanément ou non d'une ou plusieurs fonctions d'un ou plusieurs processus ou d'un ou plusieurs organismes.

L'invention a donc aussi pour objet un support présentant une série d'emplacements pour la mise en oeuvre d'une méthode de l'invention, caractérisé en ce que chacun desdits emplacements permet la détection et/ou la mesure d'une variation de fonction.

La substance à cribler peut être présente dans les puits ou être ajoutée en fin de réaction.

D'autres avantages et caractéristiques de l'invention apparaîtront dans les exemples de réalisation de l'invention, qui suivent et qui se réfèrent aux dessins en annexe dans lesquels :
La figure 1 est une représentation de la mise en évidence d'inhibiteur de protéinase à acide aspartique du virus du HIV, par le procédé de l'invention.
La figure 2 est une représentation schématique de la mise en évidence d'inhibiteur de l'épissage protéique de l'intéine de RecA de Mycobacterium tuberculosis, par le procédé de l'invention.
La figure 3 est une représentation schématique de la mise en évidence d'inhibiteurs suicides de Beta-lactamase par le procédé de l'invention.
Les figures 4, 5 et 6 représentent la comparaison de la sensibilité et de la reproductibilité des plusieurs méthodes de détection d'une fonction selon le procédé de l'invention.
La figure 7 représente les effets de différentes substances (0,4 µM final) sur la variation de d'activité de la beta-lactamase TEM-1.
La figure 8 représente les effets de différentes substances (21,5 µM final) sur la variation de d'activité de la beta-lactamase TEM-1.
La figure 9 représente la caractérisation de la résistance d'un inhibiteur.
La figure 10 représente l'effet de différentes substances (2,5 µM final) sur la variation d'activité de la protéase du virus HIV-1 à t=0 min. et t=180 min.

### Exemple I :Effets des substances A, B, C et D sur une fonction d'un échantillon.

Les tableaux ci-dessous illustrent différents résultats susceptibles d'être obtenus par le procédé de l'invention sur un échantillon comprenant une fonction. La mesure de variation de signaux se fait par toute technique connue par l'homme de métier. Les signaux sont mesurés suivant leur propriété, de manière directe ou non, qui peut nécessiter ou non la présence d'un rapporteur. Il est envisageable que le(les) signaux(s) soi(en)t lu(s) en continu sous forme de cinétique par exemple. La méthode de l'invention offre donc un grand choix de modes de réalisation à partir des fonctions et des moyens mis en oeuvre pour la (les) révéler.

On entend par variation de signaux en présence d'une substance (tableau 1) :
- Une augmentation d'activité qui peut dépendre ou non de la quantité de substance(s) ajoutée(s).
- Une diminution de l'activité qui peut dépendre ou non de la quantité de substance(s) ajoutée(s).
- Une inhibition de l'activité étudiée qui peut dépendre ou non de la quantité de substance (s) ajoutée (s).
1) Le tableau 1 ci-dessous montre que la substance A permet d'augmenter l'activité fonctionnelle. Plus A est présente en grande quantité, plus la fonction est active.

**Tableau 1.**

| | Signal |
|---|---|
| Sans substance | 0 |
| En présence d'une substance A | ++ |
| En présence de deux fois plus d'une substance A | ++++ |

2) Le tableau 2 ci-dessous montre que la substance B a le même effet sur la fonction quelque soit sa quantité.

**Tableau 2.**

| | Signal |
|---|---|
| Sans substance | 0 |
| En présence d'une substance B | + |
| En présence de deux fois plus d'une substance B | + |

3) Le tableau 3 ci-dessous montre que la substance C n'a aucun effet sur la fonction. Par contre, au delà d'un seuil 2C, la substance C a un effet négatif sur la fonction.

**Tableau 3.**

| | Signal |
|---|---|
| Sans substance | + |
| En présence d'une substance C | + |
| En présence de deux fois plus d'une substance C | 0 |

4) Le tableau 4 ci-dessous montre que la substance D inhibe la fonction.

**Tableau 4.**

| | Signal |
|---|---|
| Sans substance | + |
| En présence d'une substance D | 0 |
| En présence de deux fois plus d'une substance D | 0 |

### Exemple II : Mesure des variations d'activité des fonctions 1 et 2 en présence d'une substance.

Le procédé de l'invention permet aussi d'étudier l'effet d'une substance sur une ou plusieurs fonctions. En effet, une substance est susceptible d'agir sur le site actif de deux fonctions différentes. C'est le cas par exemple des chélateurs d'ions bivalents comme l'EDTA qui inhibent à la fois les métalloprotéinases et des polymérases (ces deux enzymes ayant besoin d'ions bivalents pour fonctionner). D'autre part, il est également possible que l'effet d'une substance sur une fonction induise des métabolites secondaires responsables de variation d'activité d'une ou plusieurs autres fonctions. Les tableaux ci-dessous illustrent ces différents résultats.
1) Le tableau 5 ci-dessous montre que la substance A permet d'augmenter l'activité d'une fonction 1 et inhibe une fonction 2.

**Tableau 5**

| | Fonction 1 | Fonction 2 |
|---|---|---|
| Sans substance | + | + |
| En présence d'une substance A | +++ | 0 |
| En présence de deux fois plus d'une substance A | ++++ | 0 |

2) Le tableau 6 ci-dessous montre que la substance A permet d'augmenter l'activité d'une fonction 1 mais peu celle de l'activité 2. En revanche une fois la fonction 1 activée, il y a activation de la fonction 2. Mais en présence d'une trop grande quantité de A, il y a suractivation de 1 ce qui induit une inhibition de 2.

**Tableau 6**

| Fonction 1 | Fonction 1 | Fonction 2 | Fonctions 1 et 2 |
|---|---|---|---|
| Sans substance | + | 0 | + |
| En présence d'une substance A | +++ | ++ | +++++ |
| En présence de deux fois plus d'une substance A | +++++ | 0 | +++++ |

### Exemple III : effets d'une combinaison de substances sur une fonction d'un échantillon.

Une autre forme de mise en oeuvre du procédé de l'invention consiste à tester plusieurs substances sur une fonction. Ces substances peuvent être ajoutées de manière combinée ou de manière séquentielle à chacune des étapes du procédé. Le test de plusieurs substances sur une fonction va permettre de déterminer si ces substances ont entre elles un effet coopératif ou un effet antagoniste, si elles sont entre elles compétitives ou complémentaires. Le procédé de l'invention permet de tester toutes les combinaisons possibles de substances sur une ou plusieurs fonctions.
1) Le tableau 7 ci-dessous montre que la substance A augmente l'activité fonctionnelle. La substance B est sans effet sur la fonction. Le couple de substances A et B augmente encore plus que la substance A seule l'activité de la fonction. La substance B un effet complémentaire ou coopératif de la substance A.

**Tableau 7**

| | Signal |
|---|---|
| Sans substance | 0 |
| En présence d'une substance A | + + |
| En présence d'une substance B | 0 |
| En présence des substances A et B | + + + + |

2) Le tableau 8 ci-dessous montre que l'activité fonctionnelle est augmentée de la même façon en présence de la substance A seule ou des substances A et B. La substance B n'a donc aucun effet sur la fonction, comme le montre le test fonctionnel en présence de la substance B seule.

**Tableau 8**

| | Signal |
|---|---|
| Sans substance | 0 |
| En présence d'une substance A | + + + + |
| En présence d'une substance B | 0 |
| En présence des substances A et B | + + + + |

3) Le tableau 9 ci-dessous montre que les substances A et B séparément et le couple de substances A et B inhibent la fonction.

**Tableau 9**

| | Signal |
|---|---|
| Sans substance | + |
| En présence d'une substance A | 0 |
| En présence d'une substance B | 0 |
| En présence des substances A et B | 0 |

4) Le tableau 10 ci-dessous montre que les substances A et B séparément n'ont pas d'action sur la fonction, alors que le couple de substances A et B augmentent l'activité de la fonction.

**Tableau 10**

| | Signal |
|---|---|
| Sans substance | 0 |
| En présence d'une substance A | 0 |
| En présence d'une substance B | 0 |
| En présence des substances A et B | +++ |

### Exemple IV : effets d'une combinaison de substances sur une ou plusieurs fonctions.

Les variations d'une ou plusieurs fonctions obtenues en présence d'une ou plusieurs substances peuvent être comparées entre elles pour déterminer la ou les meilleure(s) substance(s) ou la meilleure combinaison de substances et ainsi que la meilleure quantité de chaque substance.

Le procédé de l'invention peut donc également être mis en oeuvre pour étudier les différentes formes galéniques d'une ou plusieurs substances. Après avoir identifié une ou plusieurs substances capables de modifier l'activité d'une ou plusieurs fonctions, ces substances seront utilisées pour traiter un produit. La forme galénique de la ou des substances sera importante pour une efficacité optimale de la ou desdites substances sur la ou les fonctions du ou desdits produits. C'est pourquoi le ou les composants de la forme galénique pourront être ajoutés à la ou aux dites substances dans le procédé de l'invention pour étudier leur impact sur la variation d'activité d'une ou plusieurs fonctions.

### Exemple V : suivi de l'efficacité d'une ou plusieurs substance(s) sur une ou plusieurs fonction(s) d'un ou plusieurs organisme(s) ou processus.

Le procédé de l'invention permet également de suivre le comportement d'une ou plusieurs fonctions en présence d'une ou plusieurs substances.

En d'autres termes il permet d'étudier l'efficacité d'une ou plusieurs substance(s) (tableau 11). En soumettant une ou plusieurs fonctions d'un organisme ou d'un processus à une ou plusieurs substances, on peut induire des variations de la ou des dites fonctions, qui font qu'elles n'auront plus le même comportement vis à vis d'une ou plusieurs substances. En traitant une ou plusieurs fonctions par une ou plusieurs substances, on la soumet en quelque sorte à une pression de sélection. Cette ou ces fonctions pourront par exemple devenir tolérantes vis à vis d'une ou plusieurs substances ou elles pourront présenter des phénomènes de résistances. Ainsi l'effet d'un traitement d'une ou plusieurs fonctions d'un organisme ou processus à une ou plusieurs substances, pourra être testé par le procédé de l'invention en mesurant pour une ou plusieurs fonctions leur variation d'activité en présence de la ou des dites substances. Cette mise en oeuvre particulière du procédé de l'invention permettra aussi d'évaluer la toxicité d'une ou plusieurs substances sur une ou plusieurs fonctions d'un organisme ou processus.

Il s'agit donc par exemple d'un suivi de l'évolution d'une (ou plusieurs) fonctions lorsqu'elles sont soumises à la présence d'une (ou plusieurs) substances.

Le tableau 11 ci-dessous illustre ce type de résultat sur la toxicité d'une substance A sur les fonctions 1 et 2. Le tableau 11 montre au temps t, qu'une substance A a augmenté l'activité d'une fonction 1, qui continue d'augmenter au temps t+1. Par contre, au temps t+2, la substance A a perdu son efficacité sur la fonction 1. La fonction 1 devient donc moins sensible à l'effet de la substance A avec le temps. Le tableau 11 montre aussi que la substance A inhibe de plus en plus avec le temps une fonction 2.

**Tableau 11**

| | Fonction 1 | Fonction 2 |
|---|---|---|
| En absence de substance | + | +++ |
| Mesure au temps t en présence de la substance A | ++ | ++ |
| Mesure au temps t+1 en présence de la substance A | ++++ | + |
| Mesure au temps t+2 en présence de la substance A | + | 0 |

Le procédé de l'invention permet donc d'apprécier dans le temps l'efficacité de la ou des meilleures substances capables de faire varier au mieux la ou les activités de la ou desdites fonctions.

### Exemple VI : Criblage à haut débit d'inhibiteurs de protéinase à acide aspartique.

Parmi les protéines virales nécessaires au cycle de multiplication du virus du HIV, on peut noter la protéinase à acide aspartique, utilisée comme cible dans différentes thérapies antivirales (comme par exemple la trithérapie). La recherche d'inhibiteurs puissants de protéinases à acide aspartique est en pleine expansion compte tenu de l'adaptation du virus aux inhibiteurs : 6 semaines après le début d'une thérapie avec un inhibiteur de protéinase de HIV-1 (indinavir), des isolats résistants à l'inhibiteur sont détectés. Ils se manifestent entre autre par une mutation au niveau du résidu V82A/F/T de la protéinase de HIV-1 (4). L'objectif de différents laboratoires pharmaceutiques est donc de développer un système de criblage à haut débit pour trouver de nouveaux inhibiteurs de protéinase à acide aspartique.

Le test de criblage de nouveaux inhibiteurs de protéinase à acide aspartique (HIV) suivant repose sur le procédé de l'invention. Le gène cible codant pour la protéinase à acide aspartique sauvage de HIV est placé sous contrôle du promoteur et du terminateur de l'ARN polymérase du phage T7, ce qui permet de le transcrire et de traduire la protéine correspondante *in vitro.*

La molécule rapporteur utilisée à l'étape (d) est un peptide contenant des sites de coupure spécifiques de la protéinase de HIV. Ce peptide est lié covalemment respectivement en N et C terminal à une molécule fluorescente (comme par exemple la fluoresceïne) et à un quencher de cette dernière (comme par exemple la rhodamine).

Le gène préparé à l'étape (a) est placé dans différents puits d'une plaque de microtitration. Les puits correspondant sont chargés avec un mélange de réaction de transcription et de traduction concentré couplé (composants nécessaires à la transcription et à la traduction concentrés) contenant la molécule rapporteur. Les plaques correspondantes sont rapidement congelées pour éviter l'initiation précoce de la réaction de transcription et de traduction. Cette réaction de transcription et de traduction peut être soit réalisée avec un extrait cellulaire de traduction de *E*. *coli,* soit avec un extrait de traduction de cellules eucaryotes. L'extrait de traduction choisi dépend du type d'amorces utilisées en (a) avec ou sans site de fixation des ribosomes. Les plaques congelées sont placées sur un automate, qui dépose dans chaque puits un volume de la ou des substance(s) à tester permettant de diluer le mélange de transcription et de traductionjusqu'à ce qu'il soit une fois concentré.

Si cette substance est active en tant qu'inhibiteur de la protéine cible, elle empêche la protéinase à acide aspartique de HIV d'attaquer la molécule rapporteur. La fluoresceïne et la rhodamine se trouvent alors dans un environnement suffisamment proche pour assurer un transfert d'énergie de type FRET (fluorescence resonance energy of transfert). Après exposition du peptide rapporteur à une longueur d'onde donnée (environ 490 nm), seule la fluorescence rouge du quencher sera détectable.

Si la substance testée n'est pas capable d'inhiber la protéinase à acide aspartique, le peptide rapporteur est clivé. La fluoresceïne et la rhodamine sont alors trop éloignées pour assurer un transfert d'énergie. Après exposition des produits de clivage à une longueur d'onde donnée (environ 490 nm), seule la fluorescence verte de la fluoresceïne est détectable.

Lorsqu'une substance inhibitrice de la protéinase à acide aspartique est découverte, un test de contrôle de spécificité est réalisé en incubant cette substance dans une réaction de transcription et de traduction permettant l'expression d'un autre gène rapporteur (type GFP) pour vérifier que cette substance ne soit pas en fait un inhibiteur de la transcription ou de la traduction.

A chaque fois qu'un nouvel inhibiteur de la protéinase est découvert, une série de tests complémentaires sont réalisés sur des réactions de transcription et de traduction permettant l'expression de protéases résistantes aux anti-protéases. Ainsi, la substance découverte peut être rapidement caractérisée afin de savoir si elle est capable d'inhiber les protéases des souches de virus actuellement résistantes aux anti-protéases.

Une représentation du procédé de l'invention est schématisée en figure 1.

### Exemple VII : Criblage à haut débit d'inhibiteur de l'épissage protéique de l'intéine de RecA de Mycobacterium tuberculosis.

Davis et al., (1992) montrent que l'excision de l'intéine de RecA de Mycobacterium tuberculosis est indispensable à l'activité de cette protéine (1). Les intéines sont des introns protéiques récemment découverts à l'intérieur de séquences protéiques. Elles possèdent toute l'information nécessaire à leur propre excision. Ce processus récemment mis en évidence est comparable à l'épissage des ARN prémessagers. Ainsi, par convention, la séquence protéique interne est appelée intéine et les deux séquences externes, extéines (3). Ce phénomène d'excision protéique spécifique est très rapide. Ces intéines ont été découvertes dans des groupes de gènes très différents et elles sont à la fois présentes chez les procaryotes et les eucaryotes.

La protéine RecA de *Mycobacterium tuberculosis* contient un tel élément. D'autres *Mycobactéries* pathogènes peuvent posséder de telle séquence dans RecA comme *Mycobacterium Leprae.* Néanmoins, ces deux introns protéiques sont différents en taille, en séquence et dans leur localisation, ce qui les rend très spécifiques (2).

En inhibant l'épissage protéique de l'intéine de Mycobacterium *tuberculosis,* on inhibe l'activité de la protéine RecA et donc la multiplication de cet organisme pathogène. Dans le cas présent, l'inhibition de l'épissage représente une nouvelle voie thérapeutique contre la multiplication de la bactérie responsable de la tuberculose. Seul un test *in vitro,* comme celui qui est proposé, permet d'analyser l'inhibition d'un phénomène de l'épissage protéique, trop rapide pour pouvoir être analysé *in vivo.*

Un système de criblage d'inhibiteurs de l'épissage de l'intéine de RecA de *Mycobacterium tuberculosis* est mis au point. Le test proposé repose sur le procédé de l'invention et consiste à exprimer in vitro le gène codant pour une protéine rapporteur telle que la GFP, au sein duquel l'intéine du gène recA Mycobacterium tuberculosis a été insérée. Avant toute réaction d'expression une substance d'une banque à intérêt pharmacologique est ajoutée au mélange de transcription et de traduction. Si cette substance est active en tant qu'inhibiteur, elle empêche l'épissage de l'intéine après la traduction, ce qui inhibe l'expression de la GFP mature. Au contraire si cette substance est inactive, l'activité de la GFP peut être détectée (figure 2).

Pour chaque substance ayant une activité inhibitrice du l'épissage des intéines, un second test permet de vérifier que cette substance est spécifique. Ce deuxième test consiste à incuber ladite substance dans un mélange de transcription et de traduction contenant le gène gfp sans aucune intéine et à vérifier que le gène peut être transcrit et traduit en présence de cette substance.

L'automatisation de ce criblage peut être réalisée suivant les mêmes conditions précédentes.

Les substances ainsi détectées pourront être testées sur d'autre intéines présentant un intérêt dans un processus donné.

### Exemple VIII : Criblage à haut débit d'inhibiteurs de beta-lactamase.

Les beta-lactamines (pénicillines et céphalosporines) représentent la classe la plus utilisée des antibiotiques en thérapie anti-infectieuse. Depuis l'introduction en thérapie de ces molécules, de nouvelles résistances à ces composés se développent. Parmi les différents mécanismes de résistances acquis par les germes résistants, un des plus importants consiste à produire une enzyme (de type beta-lactamase) capable d'hydrolyser la molécule d'antibiotique avant que celle-ci n'ait pu agir. Suite à l'utilisation mondiale et intensive des antibiotiques, la vitesse d'apparition de nouvelles résistances est actuellement plus rapide que la découverte de nouveaux antibiotiques. D'autre part, il existe peu d'inhibiteurs "suicide" de beta-lactamases (l'acide clavulanique (en association avec l'amoxicilline dans l'Augmentin), le sulbactam et le tazobactam) sur le marché, et déjà, des résistances à ces nouveaux composés ont pu être mises en évidence dans les milieux hospitaliers. L'objectif est donc de développer un système de criblage à haut débit pour trouver de nouveaux inhibiteurs de la beta-lactamase TEM-1.

Le criblage de nouveaux inhibiteurs suicide consiste à réaliser une réaction de transcription et de traduction permettant l'expression de la beta-lactamase TEM-1. Le gène bla ést sous le contrôle des séquences promotrices et terminatrices de l'ARN polymérase du phage T7. Après cette réaction de transcription de traduction, une substance issue d'une banque de molécules à intérêt pharmacologique est ajoutée au mélange réactionnel. Si cette substance est active en tant qu'inhibiteur de beta-lactamase, elle inhibe l'activité catalytique de l'enzyme. Dans l'autre cas, une beta-lactamase active est produite. L'activité beta-lactamase est alors évaluée par l'intermédiaire d'un substrat chromogénique de la beta-lactamase : la nitrocéphine.

L'automatisation de ce criblage peut être réalisée suivant les mêmes conditions décrites précédemment .

Lorsqu'une substance inhibitrice a été identifiée, une série de tests complémentaires est réalisée afin de tester l'efficacité de la molécule découverte sur l'ensemble des mutants de beta-lactamase connus comme résistants aux inhibiteurs. Pour cela l'activité des différents variants de cette beta-lactamase exprimés *in vitro* selon le procédé de l'invention est évaluée en présence de la substance inhibitrice, et le test d'activité de ces beta-lactamases est ensuite réalisé comme décrit ci-dessus.

### Exemple IX : Comparaison de plusieurs tests fonctionnels mis en oeuvre dans le procédé selon l'invention.

### 1) Comparaison de plusieurs tests fonctionnels.

La méthode de l'invention permet de comparer la sensibilité et la reproductibilité de plusieurs types de tests fonctionnels en un temps très court. Dans cette expérience, la beta-lactamase TEM-1 a été exprimée *in vitro* en utilisant 0,5 *µ*g de mRNA de ce gène et en traduisant cette enzyme comme décrit par Zubay dans un volume final de 100 *µ*l.

### a) Test 1 : dosage de l'hydrolyse de la nitrocéphine.

Différents volumes (5, 10, 15, 20, 25, 30*µ*l) de ce mélange de traduction ont été incubés à 37°C dans un volume final de 250 *µ*l de tampon de révélation de l'activité (concentration finale : NaP 40mM pH 7.0, 100*µ*g/ml de nitrocéphine et 0,25 mM DMSO). La réaction a été suivie par spectrophotométrie à 486 nm, et les valeurs d'activité obtenues ont été exprimées en pourcentage de l'activité maximale obtenue. Ces mesures ont été faites en triple exemplaires afin d'évaluer la reproductibilité de la technique de révélation.

### b) Test 2 : dosage de l'hydrolyse de l'ampicilline par révélation directe

Différents volumes (5, 10, 15, 20, 25, 30*µ*l) du mélange de traduction ont été incubés à 37°C dans un volume final de 250 *µ*l de tampon de révélation de l'activité (concentration finale : NaP 35mM pH 7.5, et 160*µ*g/ml d'ampicilline). La réaction a été suivie par spectrophotométrie à 235 nm, et les valeurs d'activité obtenues ont été exprimées en pourcentage de l'activité maximale obtenue. Ces mesures ont été faites en triple exemplaires afin d'évaluer la reproductibilité de la technique de révélation.

c) Test 3 : dosage de l'hydrolyse de l'ampicilline par révélation par fluorescence (Chen K.C. et Holmes K.K., 1986, Enhancement of fluorescence development of end products by use of a fluorescence developer solution in a rapid and sensitive fluorescent spot test for specific detection of microbial beta-lactamases, J. Clin. Microbiol., 23(3), 539-544).

Différents volumes (5, 10, 15, 20, 25, 30*µ*l) du mélange de traduction ont été incubés à 25°C pendant 15 minutes dans un volume final de 210 *µ*l de tampon I (concentration finale : 200pg/ml d'ampicilline). 40 *µ*l de tampon II de révélation ont été ajoutés (tampon II : sodium tartrate 0.78 M pH 4.5, formaldéhyde 12%), et la réaction a été incubée 10 minutes à 45°C. La lecture a été faite par fluorimétrie avec une longueur d'onde d'excitation de 365 nm, et une longueur d'émission de 430 nm, et les valeurs d'activité obtenues ont été exprimées en pourcentage de l'activité maximale obtenue. Ces mesures ont été faites en triple exemplaires afin d'évaluer la reproductibilité de la technique de révélation. Un témoin a été fait sans enzyme.

### d) Conclusions.

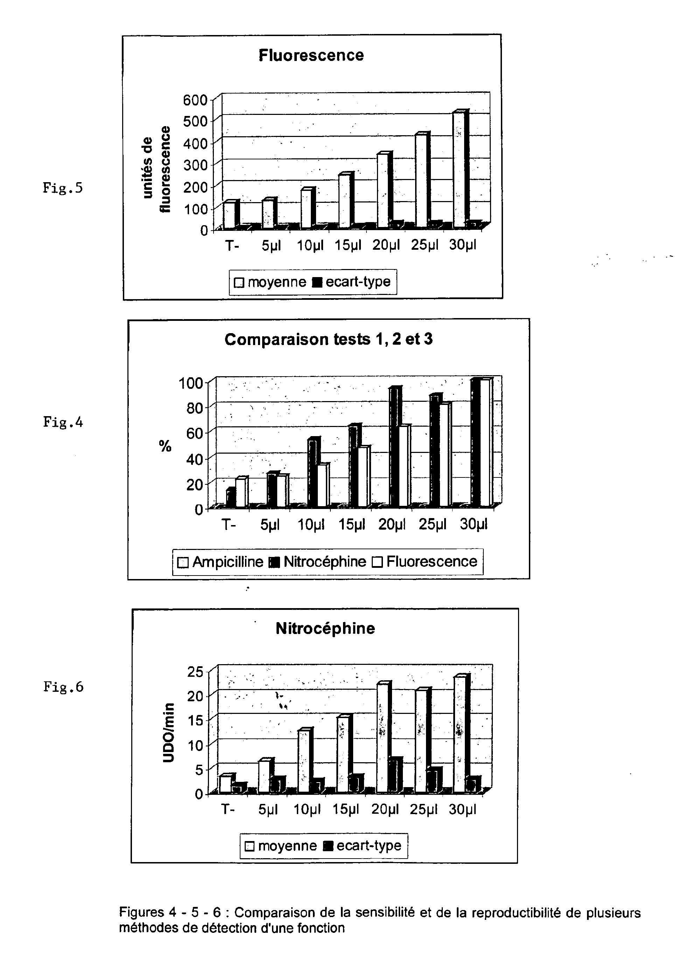

Les figures 4, 5 et 6 illustrent la comparaison de la sensibilité et de la reproductibilité de plusieurs méthodes de détection d'une fonction

La figure 4 indique que le test 2 d'activité (ampicilline) ne permet pas de révéler l'activité de l'enzyme quelque soit la concentration de celle-ci. En revanche, les tests 1 et 3 permettent d'obtenir un signal qui est corrélé au volume de traduction ajouté dans le test. D'autre part, le test d'activité 3 (fluorescence) semble donner une réponse moins sensible que le test d'activité 1 (nitrocéphine) pour des faibles concentrations en enzyme. Mais, les figures 5 et 6 indiquent que le test 3 est plus reproductible que le test 1. Le test 3 peut donc être considéré comme un test de choix pour mesurer l'activité de cette fonction beta-lactamase.

Le procédé de l'invention permet donc de tester et de valider en très peu de temps la sensibilité de plusieurs méthodes de détection d'une fonction. Ce type d'application est particulièrement intéressant dans le cadre de la mise au point d'un système de criblage qui nécessite souvent le test de sensibilité le plus précis et le plus reproductible possible, afin de pouvoir détecter la variation d'une fonction par ce test lors de l'ajout d'une substance.

### 2) Criblage moléculaire de substances pouvant avoir une activité sur une cible.

### a) Criblage de nouveaux inhibiteurs de TEM-1

Les β-lactamines (pénicillines et céphalosporines) représentent la classe d'antibiotiques la plus utilisée en thérapie anti-infectieuse. Depuis l'introduction en thérapie de ces molécules, de nouvelles résistances à ces composés se développent, favorisant l'expansion des infections nosocomiales. Parmi les différents mécanismes de résistance acquis par les germes, un des plus importants consiste à produire une enzyme (β-lactamase TEM-1 pour certaines entérobactéries par exemple, ou beta-lactamase PSE pour *Pseudomonas aeruginosa)* capable d'hydrolyser l'antibiotique avant qu'il n'ait pu agir. Il existe quelques inhibiteurs de β-lactamases utilisés en synergie avec un antibiotique, mais au fur et à mesure de leur utilisation de nouvelles formes de β-lactamases résistantes à ces inhibiteurs sont apparues.

Un criblage de nouveaux inhibiteurs de la beta-lactamase TEM-1 a été réalisé par le procédé de l'invention. Cette enzyme a été exprimée *in vitro* comme décrit par Zubay en utilisant 300*µ*g de mRNA dans un volume final de traduction de 5 ml. Chaque puit d'une plaque de microtitration a été rempli avec 5 *µ*l d'une solution d'un inhibiteur potentiel (dont l'acide clavulanique et le sulbactam) de façon à ce que cet inhibiteur soit à la concentration de 0.4*µ*M dans le volume final du test. Puis 3*µ*l du mélange de traduction ont été ajoutés dans chaque puit de la plaque de microtitration et le mélange a été incubé 3 minutes à 25°C. Le volume a alors été complété à 58*µ*l avec une solution de révélation (concentration finale : NaP 40mM pH 7.0, 100*µ*g/ml de nitrocéphine et 0.25 mM DMSO). La réaction a été suivie par spectrophotométrie à 486 nm. Trois témoins ont été réalisés : un sans extrait de traduction (donc sans enzyme), un avec le substrat seul, et un sans aucun inhibiteur potentiel.

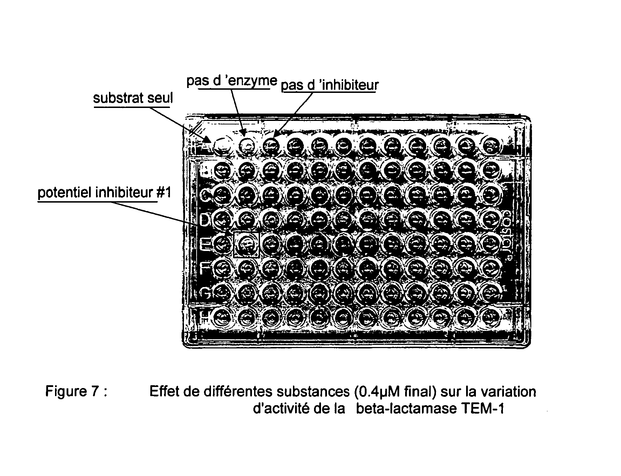

Il apparaît sur la figure 7 que seule la substance qui a été ajoutée dans le puit E2 a inhibé l'activité de la beta-lactamase TEM-1.

En mesurant l'activité résiduelle du puit E2 et en le comparant au témoin sans inhibiteur, il apparaît que cet inhibiteur potentiel#1 (qui est en fait de l'acide clavulanique) diminue de 52% l'activité de TEM-1.

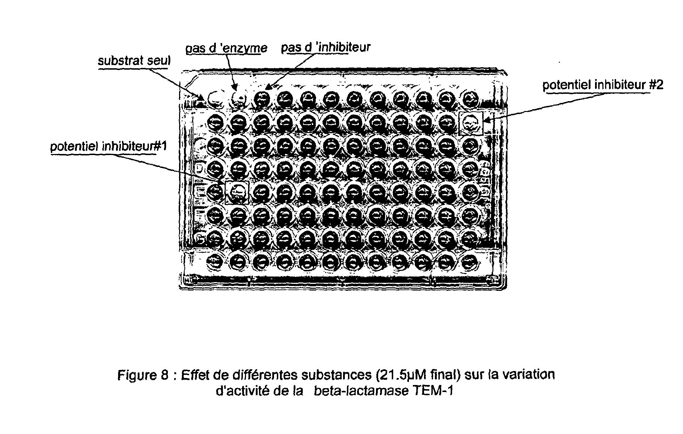

Une expérience similaire a été réalisée avec une concentration finale en inhibiteurs potentiels de 21,5 *µ*M. Il apparaît alors sur la figure 8 que l'inhibiteur potentiel #1 est toujours actif, et qu'un nouvel inhibiteur potentiel #2 est révélé dans le puit B12. Un dosage de l'activité résiduelle dans le puits E2 et B12 indique que l'inhibiteur #1 à 21.5*µ*M diminue l'activité de TEM-1 de 80%, contre 73% pour l'inhibiteur #2 (qui est en fait du sulbactam).

Après ce premier type de criblage, il est envisageable de caractériser le ou les inhibiteurs potentiels sur des cibles équivalentes (autres beta-lactamases par exemple) ou sur des mutants de la cible initiale (mutants de la beta-lactamase TEM-1 résistants aux inhibiteurs de beta-lactamases).

Cette caractérisation a été réalisée pour l'inhibiteur potentiel #1 (le plus efficace selon les expériences précédentes) sur deux mutants de la beta-lactamase TEM-1 décrits comme étant résistants à un inhibiteur de beta-lactamase. Pour cela 5 *µ*l d'un mélange de traduction exprimant soit l'enzyme sauvage, soit un des deux mutants ont été incubés séparément à 25°C pendant 3 minutes dans un volume final de 500 *µ*l de tampon I (concentration finale : NaP 50mM pH 7.0, 1.5 *µ*M d'inhibiteur potentiel #1). Puis 100*µ*g/ml de nitrocéphine et 0.25 mM final de DMSO ont été rajoutés, et la réaction a été suivie par spectrophotométrie à 486 nm. Les résultats obtenus ont été exprimés en pourcentage de l'activité maximale mesurée sur l'enzyme en absence d'inhibiteur.

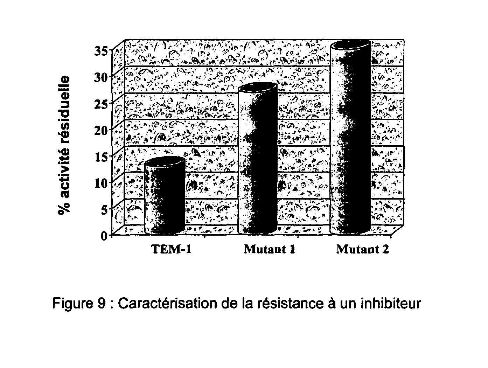

La figure 9 indique que dans ces conditions, l'enzyme sauvage TEM-1 est inhibée à 87%, alors que les deux mutants conservent respectivement une activité résiduelle de 26% et 35%. Si l'inhibiteur #1 semble efficace contre l'enzyme sauvage, il apparaît qu'il ne l'est pas contre des enzymes qui sont déjà résistantes à un inhibiteur de beta-lactamase.

Cette expérience est réalisable avec tout autre type de cible (protéase du virus HIV, récepteurs, etc...). Le procédé de l'invention permet donc de mettre en place très rapidement un criblage au niveau moléculaire de substances capables de modifier l'activité d'une cible, puis de valider les résultats sur des cibles équivalentes.

### b) Criblage de nouveaux inhibiteurs de la protéase du virus HIV-1

Un criblage de nouveaux inhibiteurs de la protéase du virus HIV-1 a été réalisée par le procédé de l'invention. Cette enzyme a été exprimée *in vitro* comme décrit par Zubay en utilisant 150*µ*g de mRNA dans un volume final de traduction de 1 ml. Chaque puit d'une plaque de microtitration a été rempli avec 60*µ*l d'un mélange contenant 10 *µ*l du mélange de traduction, 30 *µ*l de tampon (2M NaCl, 12 mM EDTA, 200 mM acétate de sodium, 2 mM DTT et 20% DMSO), 1 *µ*l à 600*µ*M du peptide substrat BACHEM M1865 (peptide DABCYL- -Abu-Ser-Gln-Asn-Tyr-Pro-Ile-Val-Gln-EDANS) et 2.5 *µ*M final de différents inhibiteurs potentiels. Ce peptide FRET a pour propriété de libérer de la fluorescence lorsqu'il est clivé par la protéase du virus HIV. Une substance qui n'inhibe pas l'activité de la protéase HIV se caractérise donc par l'apparition d'un signal fluorescent lorsque le puit est exposé aux UV. Trois témoins ont été réalisés : un sans extrait de traduction (donc sans enzyme), un avec le substrat seul, et un sans aucun inhibiteur potentiel.

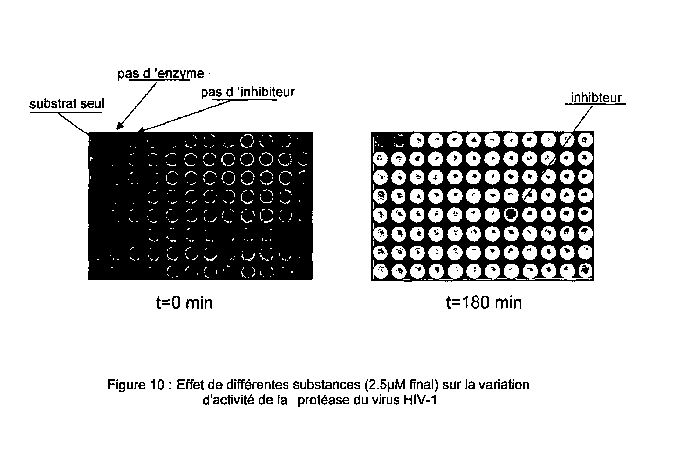

Il apparaît sur la figure 10 que seule la substance qui a été ajoutée dans le puit E8 (pepstatine A) a inhibé l'activité de la protéase de HIV.

Le procédé de l'invention permet donc de réaliser très simplement un criblage *in vitro* de substance capables de modifier l'activité d'une fonction.

Ces expériences sont réalisables avec tout autre type de cible (protéase du virus HIV, récepteurs, etc). Le procédé de l'invention permet donc de mettre en place très rapidement un criblage au niveau moléculaire de substances capables de modifier l'activité d'une cible, puis de valider les résultats sur des cibles équivalentes.

### RÉFÉRENCES BIBLIOGRAPHIQUES

1 - Davis, E.O., Jenner, P.J., Brooks, P.C., Colston, M.J., Sedgwick, S.G. (1992). Protein epissage in the maturation of M. tuberculosis RecA protein. A mechanismm for tolerating a novel class of intervening sequence. Cell 71, 201-210.
2 - Davis, E.O., Thangaraj, H.S., Brooks, P.C. et Colston, M.J. (1994). Evidence of selection for protein introns in the recAs of pathogenic mycobacteria. EMBO J. 13 (4), 699-703.
3 - Perler, F.B., Davis E.O., Dean, G.E., Gimble, F.S., Jack, W.E., Neff, N., Noren, C.J., Thorner, J. et Belfort, M. (1994). Protein epissage elements : inteins and exteins - a definition of terms and recommended nomenclature. Nucl. Acids Research 22, 1125-1127.
4 - Vasudevachari, M.B., Zhang, Y.M., Imamichi, H., Imamichi, T., Falloon, J. et Salzman, N.P. (1996). Emergence of protease inhibitor resistance mutations in human immunodeficiency virus type 1 isolates from patients and rapid screening procedure for their detection. Antimicrob Agents Chemother 40 (11), 2535-2541.
5- Chen K.C. et Holmes K.K., 1986, Enhancement of fluorescence development of end products by use of a fluorescence developer solution in a rapid and sensitive fluorescent spot test for specific detection of microbial beta-lactamases, J. Clin. Microbiol., 23(3), 539-544.
6- Gurevich V.A., Pokrovskaya I.D., Obukhova T.A. et Zozulya S., 1991, Preparative in vitro mRNA synthesis using SP6 and T7 polymerases, Anal. biochem., 195, 207-213
7- Zubay G., 1973, In vitro synthesis of protein in microbial systems, Ann. Rev. Genet., 7, 267-287

## Revendications

1. Procédé de criblage d'une substance capable de modifier la fonction connue d'une ou plusieurs protéines, **caractérisé en ce qu'**il comprend les étapes suivantes :
a) la préparation d'au moins une molécule d'acide nucléique comprenant le ou les gènes codant pour une ou plusieurs protéines et les éléments de contrôle nécessaires à la transcription et la traduction du ou desdits gènes,
b) la transcription *in vitro* dans un système acellulaire de la ou des molécules d'acide nucléique préparée(s) à l'étape (a),
c) la traduction *in vitro* dans un système acellulaire du ou des transcrit(s) préparé(s) à l'étape (b),
d) la détection et/ou la mesure de la variation d'une fonction connue soit (i) des protéines produites à l'étape (c) en présence et en absence de ladite substance ou (ii) de la substance en présence et en absence des protéines produites à l'étape (c), le système acellulaire des étapes (b) et (c) ne contenant pas ladite fonction ou s'il la contient elle n'est pas détectable dans les conditions de détection et/ou de mesure.

2. Procédé de criblage selon la revendication 1, **caractérisé en ce qu'**il comprend en outre l'étape suivante :
e) la validation sur des protéines cibles provenant d'échantillons de patients des substances générant une variation de ladite fonction.

3. Procédé de criblage selon l'une quelconque des revendications 1 ou 2, **caractérisé en ce que** ladite substance est choisie parmi des polynucléotides, des peptides, des protéines, les ions, des molécules ou des compositions chimiques naturelles ou synthétiques, des hormones, des composés aromatiques, des anticorps, des fragments d'anticorps, des gènes, des récepteurs cellulaires, des acides aminés, des glycopeptides, des lipides, des glycolipides, des sucres, des polysaccharides.

4. Procédé de criblage selon l'une quelconque des revendications précédentes, **caractérisé en ce que** ladite substance est exprimée *in vitro*

5. Procédé de criblage selon l'une quelconque des revendications précédentes, **caractérisé en ce que** ladite substance est une protéine coexprimée en même temps que la protéine cible.

6. Procédé de criblage selon l'une quelconque des revendications 1 à 5 , **caractérisé en ce que** l'étape (a) consiste (i) à préparer à partir d'un échantillon contenant des acides nucléiques plusieurs molécules d'acide nucléique chacune comprenant un fragment d'acide nucléique provenant dudit échantillon associé avec une molécule vectrice, (ii) à isoler chaque molécule d'acide nucléique constitué d'un fragment d'acide nucléique et d'une molécule vectrice.

7. Procédé de criblage selon la revendication 6, **caractérisé en ce que** la molécule vectrice associée à chaque fragment de l'étape (b) est un vecteur plasmidique ne permettant pas l'expression dudit fragment *in vivo.*

8. Procédé de criblage selon l'une quelconque des revendications précédentes, **caractérisé en ce que** ladite fonction correspond à un ensemble de protéines cibles dont les gènes codant pour ces protéines sont situés sur le même fragment d'ADN comme dans le cas d'un opéron, ou à différents endroits de l'ADN.

9. Procédé de criblage selon l'une quelconque des revendications 1 à 8, **caractérisé en ce que** la détection et/ou la mesure de la variation de fonction de la ou des protéines produites à l'étapes (c) ou de la substance est réalisée à l'étape (d) par un test fonctionnel mettant en oeuvre la présence à l'une des étapes (a), (b), (c) ou (d) d'une ou plusieurs molécules rapporteur permettant de détecter et/ou de mesurer l'activité desdites protéines ou de ladite substance.

10. Procédé de criblage selon la revendication 9, **caractérisé en ce que** la molécule rapporteur est une molécule capable de révéler directement ou indirectement l'activité d'une ou plusieurs desdites protéines ou de ladite substance.

11. Procédé de criblage selon l'une des revendications 9 ou 10, **caractérisé en ce que** la détection de la molécule rapporteur est mesurée par fluorométrie ou colorimétrie

12. Procédé de criblage selon l'une des revendications 9 à 11, **caractérisé en ce que** la molécule rapporteur est une protéine qui est produite lors de l'étape (c) conjointement avec la ou lesdites protéines.

13. Procédé de criblage selon la revendication 12, **caractérisé en ce que** le gène codant pour la molécule rapporteur est placé sous le contrôle de séquences de régulation de la transcription et de la traduction similaires à celle du ou des gènes codant pour la ou lesdites protéines, de façon à ce que le gène rapporteur soit coexprimé avec le ou lesdits gènes.

14. Un kit pour la mise en oeuvre d'un procédé de criblage selon l'une quelconque des revendications 1 à 13, **caractérisé en ce qu'**il comprend :
- les moyens de révélation de la fonction connue (i) d'une ou plusieurs protéines produites in vitro dans le système acellulaire, ou (ii) d'une substance,
- une ARN polymérase, des séquences nucléotidiques pour la préparation des molécules d'acides nucléiques comprenant le ou les gènes permettant l'expression de la ou des protéines, les quatre nucléotides triphosphates,
- les mélanges nécessaires à ladite préparation, à la transcription et à la traduction ne contenant pas ladite fonction ou la contenant mais alors elle n'est pas détectable dans les conditions de détection et/ou de mesure,
- au moins une substance,
- éventuellement des témoins.

15. Un kit pour la mise en oeuvre d'un procédé de criblage selon l'une quelconque des revendications 1 à 13, **caractérisé en ce qu'**il comprend :
a) les produits nécessaires à la préparation des molécules d'acides nucléiques comprenant le ou les gènes permettant l'expression de la ou des protéines,
b) un support comme une plaque de microtitration ou puce contenant :
- les moyens de révélation de la fonction connue (i) d'une ou plusieurs protéines produites in vitro dans le système acellulaire, ou (ii) d'une substance,
- une ARN polymérase, les quatre nucléotides triphosphates, les mélanges de transcription et de traduction ne contenant pas la fonction recherchée ou la contenant mais alors elle n'est pas détectable dans les conditions de détection et/ou de mesure,
- au moins une substance,
- éventuellement des témoins.
